# EUROPEAN PATENT APPLICATION

(11) **EP 4 494 764 A1**
(43) Date of publication of application: **22.01.2025**
(21) Application number: 23759245.6
(22) Date of filing: 23.02.2023
(51) Int. Cl.: B04B 9/14

(54) **CONTROL METHOD, MULTI-FLUX CENTRIFUGAL PLATFORM AND COMPUTER READABLE STORAGE MEDIUM**

(30) Priority: 25.02.2022 CN 202210178004
(71) Applicant: Edan Instruments, Inc., Shenzhen, Guangdong 518000 (CN)
(72) Inventor: PI, Shiwei, Shenzhen, Guangdong 518000 (CN); LI, Guo, Shenzhen, Guangdong 518000 (CN); ZHAO, Zhixiang, Shenzhen, Guangdong 518000 (CN)
(74) Representative: Herrero & Asociados, S.L.
(86) International application number: PCT/CN2023/077942
(87) International publication number: WO 2023/160623

(57) **Abstract**

Provided in the present application are a control method, a multi-flux centrifugal platform and a computer readable storage medium, relating to the technical field of molecular detection. The method comprises: acquiring the number of test cards; determining a card holder rotation policy corresponding to the number of the test cards of the multi-flux centrifugal platform; controlling the card holder to rotate according to the policy, and exposing a card position of the card holder at a card placement port. The present application determines the rotation policy corresponding to a test card balancing result by means of the number of test cards, so that a card position is exposed at the card placement port by means of rotation, a user is thus guided to place a test card on the card position of the card placement port, then the balancing of the test cards is realized, and noise caused by unreasonable placement of the test cards during centrifugal treatment is reduced. In addition, the multi-flux centrifugal platform provided by the present application operates according to the method to achieve balancing, excessive participation of the user for balancing is not needed, and the user does not need to worry about the balancing effect and operation is more intelligent.

## Description

The present application claims priority to Chinese Patent Application No. 202210178004.3, entitled "CONTROL METHOD, MULTI-FLUX CENTRIFUGAL PLATFORM, AND COMPUTER-READABLE STORAGE MEDIUM", filed on February 25, 2022, which is herein incorporated by reference in its entirety.

### TECHNICAL FIELD

The present disclosure relates to the field of molecular detection technologies, and in particular to a control method, a multi-flux centrifugal platform, and a computer-readable storage medium.

### BACKGROUND

A molecular diagnostic device utilizes a molecular diagnostic technology. The molecular diagnostic technology refers to a diagnostic technology that uses nucleic acids or proteins as biomarkers for clinical detection, which provides information and decision-making basis for prediction, diagnosis, prevention, treatment and prognosis of diseases.

When the molecular diagnostic device detects a detection card, it is necessary to centrifuge the detection card in advance. In some cases, the number of the detection cards is insufficient for even distribution on a card holder, resulting in noise during centrifugation.

### SUMMARY OF THE DISCLOSURE

In a first aspect, the present disclosure provides a control method based on a multi-flux centrifugal platform, and the control method includes:
obtaining the number of detection cards;
determining a strategy for rotating a card holder of the multi-flux centrifugal platform, wherein the strategy corresponds to the number of the detection cards; and
controlling the card holder to rotate according to the strategy, so that a card position of the card holder is exposed at a card placement port.

In a second aspect, the present disclosure provides a multi-flux centrifugal platform including:
an obtaining module, configured to obtain the number of detection cards;
a determining module, configured to determine a strategy for rotating a card holder of a multi-flux centrifugal platform, wherein the strategy corresponds to the number of the detection cards; and
a controlling module, configured to control rotation of the card holder according to the strategy, so that a card position of the card holder is exposed at a card placement port.

In a third aspect, the present disclosure provides a computer-readable storage medium storing a computer program. When the computer program is executed by a processor, a control method in the first aspect is implemented.

### BRIEF DESCRIPTION OF THE DRAWINGS

In order to more clearly describe the technical solutions in some embodiments of the present disclosure, hereinafter, the accompanying drawings that are used in the description of some embodiments will be briefly described. Obviously, the accompanying drawings in the description below are merely some embodiments of the present disclosure. For those of ordinary skill in the art, other accompanying drawings may be obtained based on these accompanying drawings without any creative efforts.
FIG. 1 is a three-dimensional structural schematic view of a molecular diagnostic device in some embodiments of the present disclosure.
FIG. 2 is an exploded schematic view of the molecular diagnostic device of FIG. 1.
FIG. 3 is an exploded schematic view of an abutting plate of FIG. 2.
FIG. 4 and FIG. 5 are structural schematic views of the first shell of FIG. 3 from different perspectives, respectively.
FIG. 6 is a structural schematic view of the abutting plate of FIG. 3.
FIG. 7 is a structural schematic view illustrating a connection between a rack and the abutting plate of FIG. 2.
FIG. 8 is a structural schematic view of a transport assembly of FIG. 2.
FIG. 9 is a structural schematic view of a transport member of FIG. 8.
FIG. 10 and FIG. 11 are structural schematic views illustrating coordination of the abutting plate and the transport assembly of FIG. 2 from different perspectives, respectively.
FIG. 12 is an exploded schematic view of a detection card detection base of FIG. 2.
FIG. 13 is an exploded schematic view of a support base of FIG. 12.
FIG. 14 is a structural schematic view of a support base body of FIG. 13.
FIG. 15 is a structural schematic view of a light detection member of FIG. 13.
FIG. 16 is a structural schematic view of a sampling chamber assembly of FIG. 13.
FIG. 17 is a structural schematic view of a light generator of FIG. 12.
FIG. 18 is a structural schematic view of a detection card in some embodiments of the present disclosure.
FIG. 19 is a cross-sectional structural schematic view of the detection card of FIG. 18 along a line L-L.
FIG. 20 is a three-dimensional structural schematic view of the detection card of FIG. 18.
FIG. 21 is a schematic view illustrating a usage process of the detection card of FIG. 20.
FIG. 22 is a flowchart of a control method based on a multi-flux centrifugal platform in some embodiments of the present disclosure.
FIG. 23 is a flowchart of a control method based on a multi-flux centrifugal platform in some embodiments of the present disclosure.
FIG. 24 is a flowchart of a control method based on a multi-flux centrifugal platform in some embodiments of the present disclosure.
FIG. 25 is a flowchart of a control method based on a multi-flux centrifugal platform in some embodiments of the present disclosure.
FIG. 26 is a structural schematic view of a multi-flux centrifugal platform in some embodiments of the present disclosure.
FIG. 27 is a block schematic view of a computer-readable storage medium in some embodiments of the present disclosure.

### DETAILED DESCRIPTION

The technical solutions in some embodiments of the present disclosure may be clearly and completely described in conjunction with accompanying drawings in some embodiments of the present disclosure. Obviously, the described embodiments are only a part of the embodiments of the present disclosure, and not all embodiments. Based on the embodiments in the present disclosure, all other embodiments obtained by those of ordinary skill in the art without creative effort are within the scope of the present disclosure.

The reference to "embodiments" in the present disclosure means that, specific features, structures, or characteristics described in conjunction with some embodiments may be included in at least one embodiment of the present disclosure. The phrase appearing in various positions in the specification does not necessarily refer to the same embodiment, nor is it an independent or alternative embodiment that is mutually exclusive with other embodiments. Those of ordinary skill in the art explicitly and implicitly understand that the embodiments described in the present disclosure may be combined with other embodiments.

The present disclosure provides a molecular diagnostic device that utilizes a molecular diagnostic technology. The molecular diagnostic technology refers to a diagnostic technology that uses nucleic acids or proteins as biomarkers for clinical detection, which provides information and decision-making basis for prediction, diagnosis, prevention, treatment and prognosis of diseases. Especially in the face of various sudden infectious diseases, the most cost-effective measure is to quickly and accurately perform molecular diagnosis.

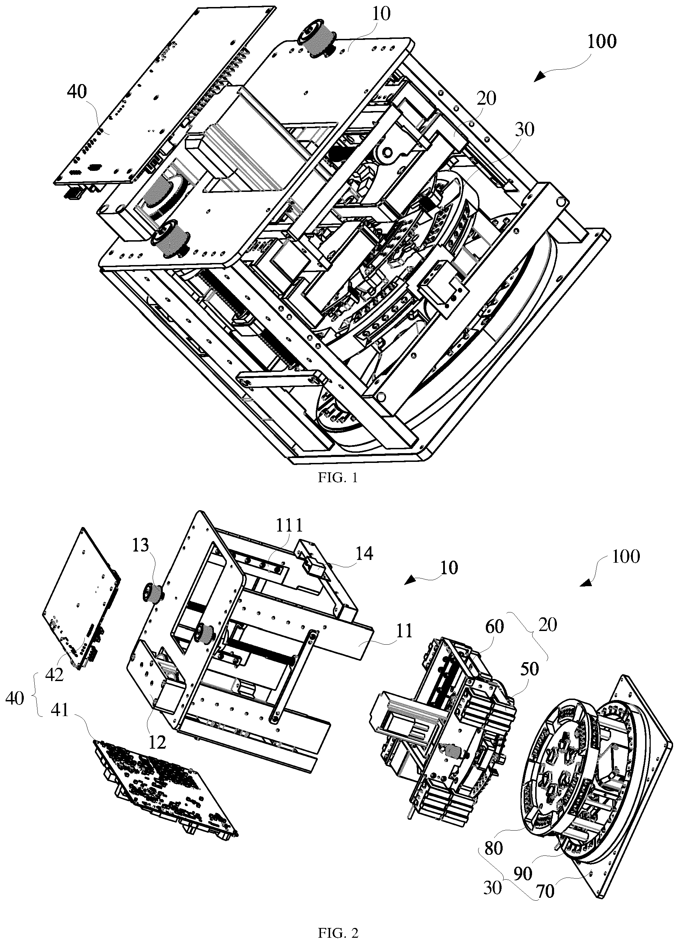

As illustrated in FIG. 1 and FIG. 2, FIG. 1 is a three-dimensional structural schematic view of a molecular diagnostic device 100 in some embodiments of the present disclosure, and FIG. 2 is an exploded schematic view of the molecular diagnostic device 100 of FIG. 1. The molecular diagnostic device 100 may include a rack 10, a detection card transport base 20 installed on the rack 10, a detection card detection base 30 installed on the rack 10, and a control circuit board 40 installed on the rack 10.

The detection card transport base 20 may be configured to allow a detection card to be placed on the detection card transport base 20. The detection card transport base 20 may slide relative to the rack 10, so that the detection card may be transported by the detection card transport base 20 to the detection card detection base 30. The detection card detection base 30 is configured to generate excitation light, so as to detect the detection card and form a detection signal. The control circuit board 40 may be configured to control sliding of the detection card transport base 20 on the rack 10, and control the detection card detection base 30 to detect the detection card, and receive and process the detection signal to form diagnostic data.

In some embodiments, the molecular diagnostic device 100 may further include an input device that may be electrically connected to the control circuit board 40, such as a display, a keyboard, or a scanning device 14 (illustrated in FIG. 2), etc. Therefore, a control instruction is input to the molecular diagnostic device 100, such as the control circuit board 40, through the input device, so that the molecular diagnostic device 100 controls the detection card transport base 20 and/or the detection card detection base 30 through the control circuit board 40.

As illustrated in FIG. 2, the rack 10 may include a rack body 11, a first driving assembly 12 installed on the rack body 11, and a lead screw 13 installed on the rack body 11. The rack body 11 is configured to install structures, such as the detection card transport base 20, the detection card detection base 30, or the control circuit board 40, etc. The first driving assembly 12 is configured to be electrically connected to the control circuit board 40, to receive control from the control circuit board 40. The first driving assembly 12 drives the lead screw 13 to rotate under the control of the control circuit board 40. The lead screw 13 is rotatably connected to the detection card transport base 20, so that under driving of the first driving assembly 12, the detection card transport base 20 slides relative to the rack body 11 in an extending direction of the lead screw 13, achieving transportation of the detection card.

The rack body 11 may form a frame structure as a whole. The interior of the rack body 11 may be configured to accommodate the detection card transport base 20 and the detection card detection base 30, and the detection card transport base 20 is located above the detection card detection base 30. In some embodiments, the detection card transport base 20 and the detection card detection base 30 may also be installed on other positions of the rack body 11, which is not repeated here.

In the present disclosure, directions, such as "up", "down", "front", "back", "left", "right", "top", "bottom", "upper", and "lower", etc., may be configured for description. It should be understood that the directions or positional relationships indicated by the terms, such as "center", "longitudinal", "transverse", "length", "width", "thickness", "up", "down", "front", "back", "left", "right", "vertical", "horizontal", "top", "bottom", "inside", "outside", "clockwise", "counterclockwise", "axial", "radial", "circumferential", etc., are based on the methods or positional relationships shown in the accompanying drawings. It is only intended to facilitate the description of the embodiments of the present disclosure and simplify the description, but does not indicate or imply that the device or the element referred to must have a specific orientation, be constructed and operated in a specific orientation. Therefore, it cannot be understood as a limitation on the embodiments of the present disclosure.

The rack body 11 is provided with a first guiding rail 111. The first guiding rail 111 may be configured to allow the detection card transport base 20 to be installed on the first guiding rail 111, so that the detection card transport base 20 slides on the first guiding rail 111 and slides in an extending direction of the first guiding rail 111. In some embodiments, the extending direction of the first guiding rail 111 may be a vertical direction.

It should be pointed out that the terms "first", "second", etc., in the present disclosure are only configured to describe and cannot be understood as indicating or implying relative importance or implicit indicating the quantity of technical features indicated. Therefore, features that are defined as "first", "second", etc., may explicitly or implicitly include one or more features.

The lead screw 13 may be installed on the rack body 11. Under the driving of the first driving assembly 12, the lead screw 13 may push the detection card transport base 20 to slide on the rack body 11, such as the first guiding rail 111, so that the detection card transport base 20 transports the detection card. In some embodiments, the extending direction of the lead screw 13 may be the same as the extending direction of the first guiding rail 111.

The rack 10, such as the rack body 11, is provided with the scanning device 14 that is electrically connected to the control circuit board 40. The scanning device 14 may be configured for image scanning to an information identifier on the detection card, such as a two-dimensional code or a bar code, etc., and transmit an image to the control circuit board 40. The control circuit board 40 recognizes the images and obtain relevant information of the detection card. The scanning device 14 may be disposed on a sliding path of the detection card transport base 20 relative to the rack 10, so that when the detection card is placed on the detection card transport base 20, the information identifier on the detection card is scanned by the scanning device 14.

As illustrated in FIG. 2, the detection card transport base 20 may include an abutting plate 50 and a transport assembly 60 that are installed on the rack 10, such as the first guiding rail 111. The transport assembly 60 is configured to allow the detection card to be placed on the transport assembly 60. The abutting plate 50 is in threaded connection with the lead screw 13, so that the abutting plate 50 slides on the first guiding rail 111 under the action of the first driving assembly 12, thereby driving the transport assembly 60 to slide on the first guiding rail 111. The transport assembly 60 may transport the detection card to the detection card detection base 30.

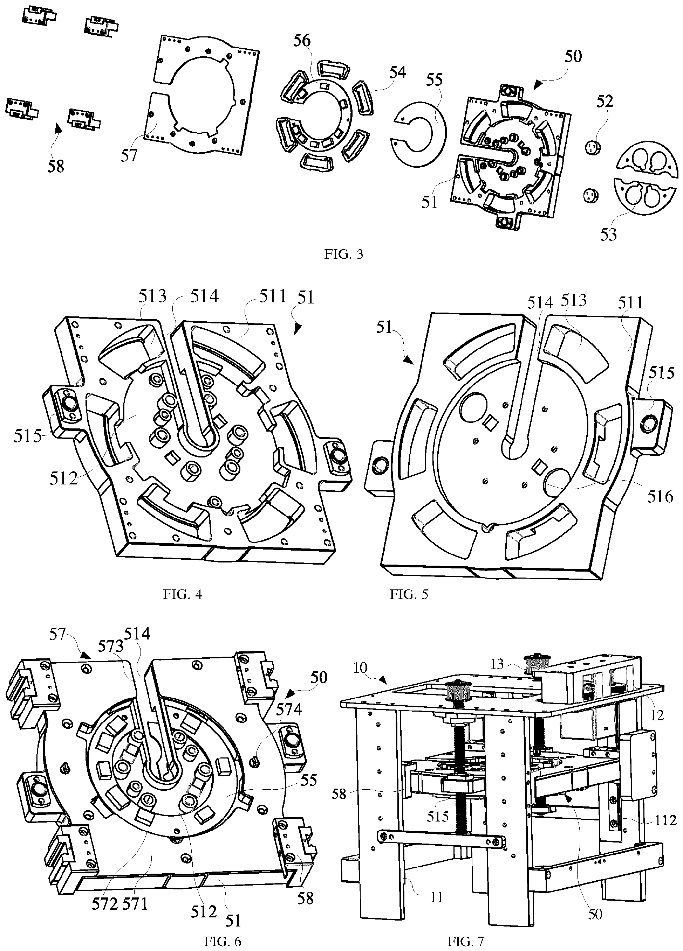

As illustrated in FIG. 3, FIG. 3 is an exploded schematic view of the abutting plate 50 of FIG. 2. The abutting plate 50 may include a first shell 51, an electromagnetic member 52, a pressing member 53, a first heating member 54, a second heating member 55, a first circuit board 56, a second shell 57, and a first sliding rail 58. The first shell 51 is installed on the rack 10, such as the lead screw 13. The electromagnetic member 52 is disposed on a side of the first shell 51 facing the detection card detection base 30. The pressing member 53 is configured for fixing the detection card on the transport assembly 60. The first heating member 54 and the second heating member 55 are disposed on the first shell 51. The first circuit board 56 is installed on the first shell 51 and electrically connected to the electromagnetic member 52, the first heating member 54, and the second heating member 55, respectively. The second shell 57 covers a side of the first shell 51 away from the electromagnetic member 52. The first sliding rail 58 is disposed on the first shell 51 and installed on the rack 10, such as the first guiding rail 111.

The first shell 51 and the second shell 57 are fastened to form an abutting plate body. The first circuit board 56 may be electrically connected to the control circuit board 40. The electromagnetic member 52 may generate a magnetic force under the control of the control circuit board 40, so that the pressing member 53 is attached to the first shell 51. The magnetic force of the electromagnetic member 52 may be eliminated under the control of the control circuit board 40, so as to avoid adhesion to the pressing member 53. The first heating member 54 and the second heating member 55 may heat the detection card under the control of the control circuit board 40. The first sliding rail 58 may slide on the rack 10, such as the first sliding rail 111. The first shell 51 is connected to the rack 10, such as the lead screw 13, so as to slide in the extending direction of the first guiding rail 111 under the rotation of the lead screw 13.

As illustrated in FIG. 4 and FIG. 5, FIG. 4 and FIG. 5 are structural schematic views of the first shell 51 of FIG. 3 from different perspectives, respectively. The first shell 51 may be made of a rigid material, such as a plastic or a metal, etc. The first shell 51 may have a plate-like structure, or the first shell 51 may have other shapes, which is not repeated here. The first shell 51 may include a shell body 511. An accommodating groove 512 is defined in a middle of a side of the shell body 511 facing the second shell 57, and configured to accommodate the first circuit board 56. A cross-section of the accommodating groove 512 may be circular or other shapes.

The shell body 511 may define a clamping hole 513 around the accommodating groove 512, and the clamping hole 513 is configured for installing the first heating member 54. The clamping hole 513 may be evenly distributed around the accommodating groove 512. The number of the clamping holes 513 may be one or more. In some embodiments, the number of the clamping holes 513 may also be one of two, three, four, five, and six, etc. In some embodiments, the number of the clamping holes 513 may be six. In some embodiments, a shape of the clamping hole 513 is a segment of a circular structure.

The shell body 511 defines a notch 514, so as to give way to the transport assembly 60. The notch 514 extends from an edge of a side of the shell body 511 close to the scanning device 14, towards the interior of the shell body 511. In some embodiments, the notch 514 is located between two adjacent clamping holes 513.

A threaded connection part 515 is disposed on a part of the shell body 511 close to the lead screw 13. The threaded connection part 515 may be sleeved on the lead screw 13. The interior of the threaded connection part 515 is provided with an internal thread that is matched with an external thread of the lead screw 13, so that the shell body 511 may move in the extending direction of the lead screw 13 under the rotation of the lead screw 13.

A middle part of a surface of the shell body 511 away from the second shell 57 may be configured to connect the electromagnetic member 52, the pressing member 53, and the second heating member 55. In some embodiments, multiple receiving grooves 516 are defined and uniformly distributed on a middle part of a surface of the shell body 511 away from the second shell 57 along the circumferential direction, and the receiving grooves 516 are configured to accommodate the electromagnetic members 52. The number of the receiving grooves 516 may be multiple, and may be one of two, three, four, five, and six, etc. In some embodiments, the number of the receiving grooves 516 may be two.

As illustrated in FIG. 3, the electromagnetic member 52 may be energized to generate the magnetic force, thereby adsorbing and fixing the pressing member 53. The electromagnetic member 52 is electrically connected to the first circuit board 56, and there may be two electromagnetic members 52. One electromagnetic member 52 may be installed in the receiving groove 516. The electromagnetic member 52 may be fixed on the shell body 511 through a connection mode, such as screwing, clamping, plugging, welding, or bonding, etc.

As illustrated in FIG. 5, the pressing member 53 may be made of the rigid material, such as the metal. In some embodiments, the pressing member 53 may be made of the metal that is able to be adsorbed by an electromagnet under the magnetic force, such as iron. In some embodiments, each clamping member 53 may also be made of the rigid material, such as the plastic or the metal, etc. When the electromagnetic member 52 adsorbs the pressing member 53, the electromagnetic member 52 may adsorb the pressing member 53.

As illustrated in FIG. 3, the first heating member 54 is installed on the side of the first shell 51, such as the shell body 511, close to the second shell 57. The interior of the first heating member 54 may be provided with a heating element, such as a heating resistor, etc. The number of the first heating member 54 may be multiple, and may be one of two, three, four, five, and six, etc. In some embodiments, the number of the first heating members 54 may be the same as the number of clamping holes 513, and the number of the first heating members 54 and the number of the clamping holes 513 may be 6.

The first heating member 54 may be disposed in the first shell 51, such as the clamping hole 513. The first heating member 54 is clamped with the first shell 51, such as the shell body 511, so as to achieve the installation of the first heating member 54 on the first shell 51.

In some embodiments, the first heating member 54 is electrically connected to the first circuit board 56, so as to control the heating element disposed in the interior of the first heating member 54.

As illustrated in FIG. 3, the second heating member 55 is installed on the side of the first shell 51, such as the shell body 511, away from the second shell 57. The second heating member 55 may form a circular structure as a whole. The second heating member 55 may be made of a thermally conductive rigid material, such as the metal. The heating element, such as the heating resistor etc., may be disposed in the interior of the second heating member 55. The second heating member 55 is installed on the side of the shell body 511 away from the second shell 57. The second heating member 55 may be fixed on a clearance groove 517 through the connection mode, such as screwing, plugging, or snap-fit connection, etc. In some embodiments, the heating element disposed in the interior of the second heating member 55 is electrically connected to the first circuit board 56, so that the heating element disposed in the interior of the second heating member 55 is controlled.

As illustrated in FIG. 3, the first circuit board 56 may have an annular structure as a whole. The first circuit board 56 is provided with an electronic element, such as a resistor, a capacitor, an inductor, etc. The first circuit board 56 is installed in the accommodating groove 512 of the shell body 511. The first circuit board 56 may be electrically connected to the heating element inside the first heating member 54, the heating element inside the second heating member 55, and the electromagnetic element 52, respectively. Therefore, the heating element inside the first heating member 54, the heating element inside the second heating member 55, and the electromagnetic element 52 may be controlled respectively.

As illustrated in FIG. 6, FIG. 6 is a structural schematic view of the abutting plate 50 of FIG. 3. The second shell 57 may include a cover body 571 that may be fastened to the first shell 51, such as the shell body 511. The cover body 571 may be connected and fixed to the first shell 51, such as the shell body 511, through the connection mode, such as screwing, plugging, snap-fit connection, welding, or bonding, etc., which is not repeated here.

The cover body 571 may be made of the rigid material, such as the plastic or the metal, etc. The cover body 571 defines a through hole 572. The through-hole 572 may correspond to the accommodating groove 512, so as to give way to the first circuit board 56, so that the first circuit board 56 is exposed. In some embodiments, the through hole 572 may be omitted, and the cover body 571 completely covers the first shell 51, such as the shell body 511.

The cover body 571 defines a notch 573. The notch 573 extends from an edge of the cover body 571 to the interior of the cover body 571, so the notch 573 is communicated with the through hole 572. The notch 573 is disposed on a part of the cover body 571 corresponding to the notch 514, so that when the cover body 571 is fastened to the shell body 511, the notch 573 is aligned with the notch 514, thereby giving way to the transport assembly 60 and allowing the transport assembly 60 to slide in the notch 573 and the notch 514.

A hanging ear 574 is disposed on a side of the cover body 571 away from the first shell 51, so that the cover body 571 is connected to the transport assembly 60 by the hanging ear 574. The hanging ear 574 is connected and fixed to the cover body 571 through the connection mode, such as screwing, plugging, snap-fit connection, welding, or bonding, etc.

As illustrated in FIG. 6, the first sliding rail 58 is disposed on the first shell 51, so as to achieve a sliding connection between the first shell 51 and the rack 10, such as the first sliding rail 111.

As illustrated in FIG. 7, FIG. 7 is a structural schematic view illustrating a connection between the rack 10 and the abutting plate 50 of FIG. 2. The abutting plate 50 is installed on the rack 10. The first sliding rail 58 is installed on the first sliding rail 111 of the rack body 11. The threaded connection part 515 is in threaded connection with the lead screw 13. When the first driving assembly 12 moves, the lead screw 13 is driven to move synchronously, and the lead screw 13 rotates relative to the abutting plate 50. Therefore, the first sliding rail 58 of the abutting plate 50 may slide on the first guiding rail 111, achieving position movement of the abutting plate 50, specifically achieving the position movement of the abutting plate 50 in a vertical direction.

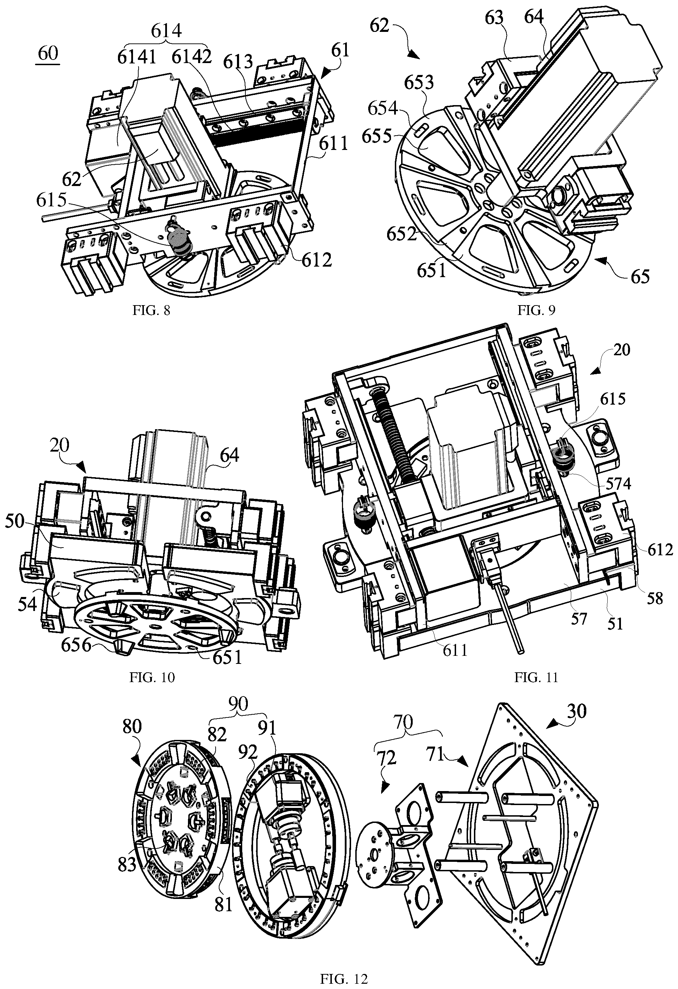

As illustrated in FIG. 8, FIG. 8 is a structural schematic view of the transport assembly 60 of FIG. 2. The transport assembly 60 may include a sliding frame 61 and a transport member 62. The sliding frame 61 is installed above the abutting plate 50 and installed on the rack 10, such as the first guiding rail 111. The transport member 62 is installed on the sliding frame 61. The sliding frame 61 may slide on the rack 10, such as the first guiding rail 111. The sliding frame 61 is connected to the abutting plate 50, so that the sliding frame 61 slides together with the abutting plate 50 on the rack 10 in certain situations. The transport member 62 may be configured to allow the detection card to be placed on the transport member 62. The transport member 62 may slide relative to the sliding frame 61. A sliding direction of the transport member 62 relative to the sliding frame 61 is different from a sliding direction of the sliding frame 61 relative to the rack 10.

It may be understood that when the transport assembly 60 is in a stretching process, the transport member 62 slides on the sliding frame 61, so that the transport assembly 60 slides to a first position outside the rack 10 and stretch in the first position. When the transport assembly 60 is in the stretched state, the detection card is placed. Then, when the transport assembly 60 is in a contracted state, the transport member 62 slides on the sliding frame 61, so that the transport member 62 slides to a second position inside the rack 10, completing the contraction. When the transport assembly 60 is in the contracted state, the transport assembly 60 may slide between a third position and a fourth position on the first guiding rail 111. In some embodiments, the transport assembly 60 may stretch in the third position. In some embodiments, the transport assembly 60 may stretch between the third position and the fourth position.

The sliding frame 61 may include a fixed frame 611, a second sliding rail 612, a second guiding rail 613, and a driving device 614. The second sliding rail 612 is installed on the fixed frame 611 and slidably connected to the rack 10, such as the first guiding rail 111. The second guiding rail 613 is installed on the fixed frame 611 and configured for installing the transport member 62. The driving device 614 is configured for driving the transport member 62 to slide on the second guiding rail 613.

The fixed bracket 611 may be made of the rigid material, such as the plastic or the metal, etc. The fixed frame 611 may have the frame structure as a whole, or the fixed frame 611 may have other shapes, which is not repeated here.

The second sliding rail 612 is disposed on the fixed frame 611, and configured to achieve a sliding connection between the fixed frame 611 and the rack 10 when the second sliding rail 612 is slidably connected to the rack 10, such as the first sliding rail 111.

The second guiding rail 613 is installed on the fixed frame 611 and configured for installing the transport member 62, so that the transport member 62 slides on the second guiding rail 613. An extending direction of the second guiding rail 613 may be consistent with the extending direction of the notch 514, so that the transport member 62 slides on the second guiding rail 613 and simultaneously slides in the notch 514.

The driving device 614 may include a second driving assembly 6141 disposed on the fixed frame 611 and a lead screw 6142 connected to the second driving assembly 6141. The second driving assembly 6141 is configured to drive the lead screw 6142 to rotate. The second driving assembly 6141 may be connected and fixed to the fixed frame 611 through the connection mode, such as a screw, a bolt, a buckle, plugging, welding, or bonding, etc. The second driving assembly 6141 may be electrically connected to the control circuit board 40 to move under the control of the control circuit board 40. The lead screw 6142 is rotatably connected to the fixed frame 611. The extending direction of the lead screw 6142 may be consistent with the extending direction of the second guiding rail 613. An outer surface of the lead screw 6142 is provided with external threads. The lead screw 6142 is in threaded connection with the transport member 62 through the external threads, so that when the lead screw 6142 rotates, the lead screw 6142 rotates relative to the transport member 62. Under the cooperation of the lead screw 6142 and the second guiding rail 613, the transport member 62 slides on the second guiding rail 613. In some embodiments, the second driving assembly 6141 may be a motor.

In some embodiments, in order to achieve that the abutting plate 50 slides together with the sliding frame 61, a traction member 615 may be disposed on the fixed frame 611. The traction component 615 may include a tension spring, for example, one end of the tension spring is connected to the fixed frame 611, and the other end of the tension spring is connected to the hanging ear 574.

As illustrated in FIG. 9, FIG. 9 is a structural schematic view of the transport member 62 of FIG. 8. The transport member 62 may include a sliding base 63, a third driving assembly 64, and a card holder 65. The sliding base 63 is installed on the second guiding rail 613 and in threaded connection with the driving device 614, such as the lead screw 6142. The third driving assembly 64 is installed on the sliding base 63. The card holder 65 is installed on the third driving assembly 64 and capable of being driven to rotate by the third driving assembly 64. The sliding base 63 slides on the second guiding rail 613, so that the sliding base 63 rotates relative to the lead screw 6142 under the driving of the driving device 614, thereby driving the third driving assembly 64 and the card holder 65 to move together to the position outside the rack 10. Therefore, the detection card may be placed on the card holder 65. The sliding base 63 may also drive the third driving assembly 64 and the card holder 65 to move to the position inside the rack 10. The third driving assembly 64 may drive the detection card on the card holder 65 to perform centrifugal motion, to complete the centrifugal processing of the detection card.

The third driving assembly 64 may be connected and fixed to the sliding base 63 through the mode, such as screwing, plugging, snap-fit connection, welding, or bonding, etc. The third driving assembly 64 may be the motor. An output shaft of the motor may be connected and fixed to the card holder 65.

The card holder 65 is disposed below the sliding base 63, and the card holder 65 is connected and fixed to the third driving assembly 64. The card holder 65 may include a card holder body 651. The card holder body 651 may be made of the rigid material, such as the plastic or the metal, etc. The card holder body 651 is a solid of revolution, and an axis of the solid of revolution is coaxial with the output shaft of the third driving assembly 64. In some embodiments, the card holder body 651 has a circular disc-shaped structure.

Multiple convex ribs 652 are disposed on a side of the card holder body 651 close to the third driving assembly 64. The convex ribs 652 radiates in all directions from a center where the card holder body 651 is connected to the third driving assembly 64. The convex ribs 652 are uniformly distributed around the part where the card holder body 651 is connected to the third driving assembly 64 along the circumferential direction.

Two adjacent convex ribs 652 and the card holder body 651 are enclosed to form a concave part 653 for accommodating the detection card. The number of the concave parts 653 may be consistent with the number of first heating members 54. In some embodiments, the number of the concave parts 653 may also be less than the number of the first heating members 54. In some embodiments, the number of the concave parts 653 may be six, and the number of the concave parts 653 may also be adjusted according to the actual situation.

A first clamping part 654 is disposed in the concave part 653 of the card holder body 651, and configured for fixing the detection card. The first fixing part 654 is located on a part of the card holder body 651 close to an edge of the card holder body 651. In some embodiments, the first clamping part 654 is a groove or a protruding column. The concave part 653 of the card holder body 651 defines a receiving hole 655, so as to accommodate the detection card. In some embodiments, the concave part 653 is a groove, and a depth of the groove may be the same as a thickness of the detection card, so that when the detection card is placed in the concave part 653, a surface of the detection card is flush with a surface of the convex rib 652 away from the card holder body 651.

The card holder body 651 may be clamped with the pressing member 53 when the pressing member 53 contacts with the pressing member 53. The clamping and fitting relationship between the card holder body 651 and the pressing member 53 may be the groove and the protruding column, plug-in structure, or buckle structure, etc. In some embodiments, in order to further enhance the fitting relationship between the card holder body 651 and the pressing member 53, an electromagnetic force connector, such as a permanent magnet or the electromagnet, etc., may be installed inside the card holder body 651 and/or inside the convex ribs 652. Therefore, the magnetic force is utilized to adsorb the pressing member 53, strengthening the fitting relationship between the card holder body 651 and the pressing member 53, so that the pressing member 53 may be in contact with the surface of the detection card to avoid unstable installation of the detection card.

In some embodiments, multiple first positioning member 656 (as illustrated in FIG. 10) are disposed on the side of the card holder body 651 away from the third driving assembly 64, so as to achieve positioning of the detection card when the detection card is transported to the detection card detection base 30, so that the detection card is placed in a predetermined position for detection. In some embodiments, each first positioning member 656 is a positioning block that extends along a direction away from the card holder body 651, and an area of a cross-section perpendicular to the extending direction of the positioning block gradually decreases. In some embodiments, the positioning block may be conical.

It may be understood that when the card holder body 651 transports the detection card to the detection card detection base 30, the transport member 62 may be located in the fourth position.

As illustrated in FIG. 10 and FIG. 11, FIG. 10 and FIG. 11 are structural schematic views illustrating coordination of the abutting plate 50 and the transport assembly 60 of FIG. 2 from different perspectives, respectively. When the abutting plate 50 is connected to the transport assembly 60, the components of the transport assembly 60 excluding the card holder 65 are first assembled together, and then the sliding frame 61 of the transport assembly 60 is placed above the abutting plate 50. The second sliding rail 612 of the sliding frame 61 is disposed on and aligned with the first sliding rail 58 of the abutting plate 50, so that the second sliding rail 612 and the first sliding rail 58 are installed together on the first sliding rail 111 of the rack 10, such as the rack body 11.

It may be understood that the abutting plate 50 and the transport assembly 60 may also be respectively installed on different first guiding rails 111. In some embodiments, only the abutting plate 50 or only the transport assembly 60 is installed on some of the first guiding rails 111.

Then, the card holder 65 of the transport member 62 of the transport assembly 60 is disposed on the side of the abutting plate 50 away from the sliding frame 61, so that the pressing member 53 is located between the first shell 51 and the card holder 65. The output shaft of the third driving assembly 64 of the transport member 62 of the transport assembly 60 passes through the notch of the abutting plate 50 and connected and fixed to the card holder 65. The hanging ear 574 of the abutting plate 50 corresponds to and is connected to the traction component 615 of the transport assembly 60.

As illustrated in FIG. 12, FIG. 12 is an exploded schematic view of the detection card detection base 30 of FIG. 2. The detection card detection base 30 may include a support frame 70 connected and fixed to the rack 10, a support base 80 installed on the support frame 70, and a detection assembly 90 installed on the support frame 70 and the support base 80. The support base 80 is located below the abutting plate 50, so as to facilitate supporting the detection card transported by the detection card transport base 20, such as the transport assembly 60. The detection assembly 90 is electrically connected to the control circuit board 40. When the detection card is placed on the support base 80, the detection assembly 90 utilizes the excitation light to perform a series of detection processes on the detection card, to generate a detection signal, and transmits the detection signal to the control circuit board 40.

As illustrated in FIG. 12, the support frame 70 has a frame structure as a whole and may be located on the bottom of the rack 10. The support frame 70 may include a base 71 and a fixed base 72. The base 71 is disposed on the bottom of the rack 10 and fixedly connected to the rack 10, and the fixed base 72 is installed on the top of the base 71.

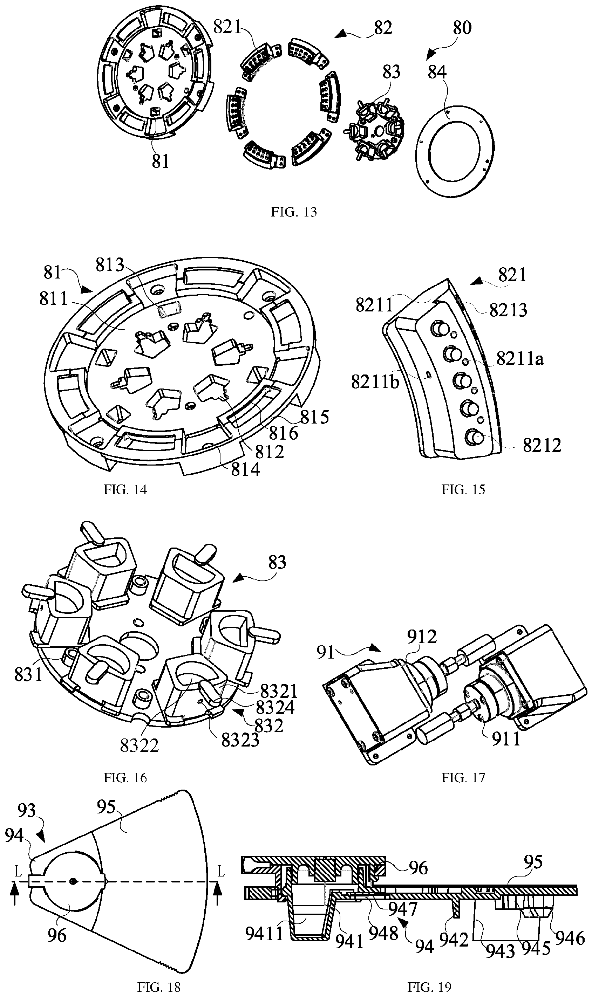

As illustrated in FIG. 13, FIG. 13 is an exploded schematic view of the support base 80 of FIG. 12. The support base 80 may include a support base body 81, a detection chamber assembly 82, a sampling chamber assembly 83, and a second circuit board 84. The support base body 81 is installed on the base 71. The detection chamber assembly 82 is installed on the support base body 81. The sampling chamber assembly 83 is installed on the support base body 81 and cooperates with the detection chamber assembly 82 to support the detection card. The second circuit board 84 is disposed on the support base body 81. The detection chamber assembly 82 may be installed on a part of the support base 80 that corresponds to the first heating member 54, so that the detection chamber assembly 82 may cooperate with the first heating member 54. The sampling chamber assembly 83 may be installed on a part of the support base 80 that corresponds to the second heating member 55, so that the sampling chamber assembly 83 may cooperate with the second heating member 55. The detection chamber assembly 82 cooperates with the sampling chamber assembly 83, so as to support and heat the detection card. The detection chamber assembly 82 may also be configured for performing excitation light detection on the detection card. The detection chamber assembly 82 and the sampling chamber assembly 83 are electrically connected to the second circuit board 84, so that heating of the detection chamber assembly 82 and heating of the sampling chamber assembly 83 may be controlled.

As illustrated in FIG. 14, FIG. 14 is a structural schematic view of the support base body 81 of FIG. 13. The support base body 81 may be made of the rigid material, such as the plastic or the metal, etc. The support base body 81 may have the plate-like structure as a whole. The top of the support base body 81 defines a placement groove 811, so as to give way to the card holder 65.

The placement groove 811 of the support base body 81 defines a clamping hole 812 configured for cooperating with the sampling chamber assembly 83. Multiple second positioning member 813 are disposed in the placement groove 811 of the support base body 81, and the second positioning member 813 cooperate with the first positioning members 656, to achieve positioning of the detection card. In some embodiments, each second positioning member 813 may be a positioning groove defined in the placement groove 811 of the support base body 81, and configured for positioning the detection card when the first positioning member 656, such as the positioning block, is placed in the second positioning member 813, such as the positioning groove. In some embodiments, a shape of the second positioning member 813, such as the positioning block, may be matched with a shape of the first positioning member 656, such as the positioning groove. It may be understood that the positioning and fitting relationship between the support base body 81 and the card holder 65 is not limited to the fitting relationship between the second positioning member 813 and the first positioning member 656. The positioning and fitting relationship between the support base body 81 and the card holder 65 may also be the magnetic force between the magnets, the magnetic force between the electromagnets, or other fitting relationships, which is not repeated here.

Multiple extending grooves 814 are uniformly distributed around the circumference of the placement groove 811 of the support base body 81. The extending groove 814 is communicated with the placement groove 811, so that in the support base body 81, an assembly table 815 is formed between two adjacent extending grooves 814. The detection card may be placed on the assembly table 815, and the extending groove 814 gives way to the detection card, so that the detection card is supported and fixed by the assembly table 815.

Each assembly table 815 of the support base body 81 defines an accommodating hole 816, so that the detection chamber assembly 82 is installed in the accommodating hole 816.

As illustrated in FIG. 13, the detection chamber assembly 82 is installed in the support base body 81, such as the accommodating hole 816. The detection chamber assembly 82 may include at least one light detection member 821, and the number of the light detection members 821 may be one of one, two, three, four, five, six, etc., so that the detection chamber assembly 82 is installed in the accommodating hole 816.

The light detection element 821 may be disposed corresponding to one first heating member 54, so that the light detection element 821 cooperates with the first heating member 54 to heat the detection card. As illustrated in FIG. 15, FIG. 15 is a structural schematic view of the light detection member 821 of FIG. 13. The light detection member 821 may include a detection base 8211 disposed in the support base body 81, such as the accommodating hole 816. The detection base 8211 may be made of the rigid material, such as the plastic or the metal, etc. The detection base 8211 extends towards the accommodating hole 816 and inserts into the accommodating hole 816. A side of the detection base 8211 close to the assembly table 815 of the support base body 81 is flush with a surface of the assembly table 815, so as to improve the aesthetic appeal of the support base 80 and configure for supporting the detection card.

Multiple excitation fibers 8212 are disposed on the detection base 8211 and distributed along an extending direction of the detection base 8211, so that each excitation fiber 8212 emits light, such as the excitation light, towards the detection card. A receiving fiber 8213 is disposed on the detection base 8211, and the extending direction of the receiving fiber 8213 and the extending direction of the detection base 8211 form an angle. The receiving fiber 8213 is configured to receive the excitation light emitted by the excitation fiber 8212 and the fluorescence formed by the excitation light illuminating the detection card.

The detection base 8211 defines multiple heating member receiving grooves 8211a, and the heating member receiving grooves 8211a are defined on the side of the detection base 8211 where the excitation fiber 8212 is disposed. The heating member receiving grooves 8211a are configured for accommodating the heating element, such as the heating resistor. The detection base 8211 may heat the detection card through the heating element. The heating element in the heating member receiving groove 8211a may be electrically connected to the second circuit board 84, so as to achieve heating under the control of the second circuit board 84.

A heating member receiving groove 8211b is defined on the side of the detection base 8211 close to the sampling chamber assembly 83, and configured for accommodating the heating element, such as the heating resistor. The detection base 8211 may heat the detection card through the heating element. The heating element in the heating member receiving groove 8211b may be electrically connected to the second circuit board 84, so as to achieve heating under the control of the second circuit board 84.

It may be understood that after the heating element is installed inside the detection base 8211, the detection base 8211 has the effect of heating the detection card. Therefore, the detection base 8211 may be referred to as a "third heating member". In some embodiments, the detection base 8211 may be integrated with the support base body 81.

As illustrated in FIG. 16, FIG. 16 is a structural schematic view of the sampling chamber assembly 83 of FIG. 13. The sampling chamber assembly 83 may include a support plate 831 fixed on the fixed base 72 and a sampling chamber installation base 832 installed on the support plate 831. The sampling chamber installation base 832 is configured to fix the detection card, and the sampling chamber installation base 832 is also configured to heat the detection card. The sampling chamber installation base 832 coordinates with the second heating member 55 to heat the detection card.

The support plate 831 may be made of the rigid material, such as the plastic or the metal, etc. The support plate 831 may have the plate-like structure as a whole, or other structures, which is not repeated here. The support plate 831 may be fixed on the fixed base 72 by screwing, plugging, bonding, or welding, etc. In some embodiments, the support plate 831 may be fixed on the fixed base 72 by screwing, plugging, bonding, or welding, etc. In some embodiments, the support plate 831 may be omitted, and the sampling chamber installation base 832 may be directly fixedly connected to the fixed base 72. In some embodiments, when the fixed base 72 is omitted, the support plate 831 may be fixed on the base 71.

The sampling chamber installation base 832 is configured to allow the detection card to be placed on the sampling chamber installation base 832. The sampling chamber installation base 832 may include multiple installation base bodies 8321. The installation base body 8321 as a whole may be made of the rigid and thermally conductive material, such as the plastic or the metal. The installation base body 8321 is configured to be placed inside the support base body 81, such as the clamping hole 812. A sampling chamber placement groove 8322 is defined on the side of the installation base body 8321 away from the support plate 831, and configured for installing the detection card.

Each installation base body 8321 defines a heating member receiving groove 8323 configured for accommodating the heating element, such as the heating resistor, so that the installation base body 8321 may heat the detection card. The heating element may be electrically connected to the second circuit board 84 for heating under the control of the second circuit board 84.

It may be understood that after the heating element is disposed inside the installation base body 8321, the installation base body 8321 has the effect of heating the detection card. Therefore, the sampling chamber installation base 832 may be referred to as a "fourth heating member".

The terms, such as "first heating member", "second heating member", "third heating member", "fourth heating member", "fifth heating member", "sixth heating member", "heating member", and "heating element", may be interchanged in some embodiments. For example, in some embodiments, the "first heating member" in other embodiments is referred to as the "second heating member", and correspondingly, the "second heating member" in other embodiments is referred to as the "first heating member".

In order to better heat the detection card, prevent condensation of water vapor from affecting the detection process, and improve the detection accuracy, a contact part 8324 is disposed on an edge of the side of the installation base body 8321 where the sampling chamber placement groove 8322 is defined, so that the contact part 8324 is in direct contact with the detection card. In some embodiments, the contact part 8324 may also be configured for positioning the detection card.

As illustrated in FIG. 13, the second circuit board 84 may be electrically connected to the heating element of the detection chamber assembly 82 and the heating element of the sampling chamber assembly 83 respectively, so as to control the heating element to heat.

The second circuit board 84 may have the annular structure and may be sleeved on the sampling chamber assembly 83. The second circuit board 84 may be directly fixed on the side of the support base body 81 close to the support frame 70 by welding, plugging, snap-fit connection, bonding, or screwing, etc. In some embodiments, the second circuit board 84 may also be directly fixed on the support frame 70, such as the fixed base 72. In some embodiments, the second circuit board 84 may also be directly fixed to the support frame 70, such as the base 71. In some embodiments, the second circuit board 84 may also be directly fixed to the sampling chamber assembly 83, such as the support plate 831.

In some embodiments, in the case that the second circuit board 84 is not configured to share a task of the control circuit board 40, the second circuit board 84 may be omitted. The heating element of the detection chamber assembly 82 and the heating element of the sampling chamber assembly 83 may be directly electrically connected to the control circuit board 40.

As illustrated in FIG. 12, the detection assembly 90 may include a light generator 91, a light receiver 92, and the detection chamber assembly 82. The light generator 91 is installed on the support frame 70, such as the base 71. The light receiver 92 is installed on the support frame 70, such as the base 71. The detection chamber assembly 82 (i.e., the detection chamber assembly 82 of the support base 80 mentioned above, the detection chamber assembly 82 may be an element shared by the support base 80 and the detection assembly 90) is installed on the support base 80, such as the support base body 81. Each of the light generator 91 and the light receiver 92 is electrically connected to the control circuit board 40. The light generator 91 is configured to generate the excitation light, and may generate the excitation light under the control of the control circuit board 40. The excitation light may be transmitted to the detection chamber assembly 82, and excite the detection card to generate the fluorescence. The fluorescence may be received by the light receiver 92. The light receiver 92 may generate the detection signal under the control of the control circuit board 40. The detection signal is transmitted to the control circuit board 40, and the control circuit board 40 processes the detection signal to generate diagnostic data.

As illustrated in FIG. 12 and FIG. 17, FIG. 17 is a structural schematic view of the light generator 91 of FIG. 12. The number of the light generators 91 may be one or more. The number of the light generators 91 may be one of two, three, four, five, and six, etc. In some embodiments, the number of the light generators 91 may be two, named as a first light generator 911 and a second light generator 912, respectively.

Each of the first light generator 911 and the second light generator 912 may be fixed on the fixed base 72 by plugging, welding, screwing, bonding, or snap-fit connection, etc. An excitation light output terminal of the first light generator 911 and an excitation light output terminal of the second light generator 912 are communicated with the excitation fiber 8212.

As illustrated in FIG. 12, the light receiver 92 is configured to be connected to the receiving fiber 8213, so that the light receiver 92 receives the fluorescence, and then transmits the detection signal that is generated by the fluorescence triggering to the control circuit board 40. The light receiver 92 is circular and disposed around the light generator 91. The light receiver 92 may include a light sensor, such as a photodiode. The light sensor, such as the photodiode, may generate an electrical signal through fluorescence irradiation.

As illustrated in FIG. 1 and FIG. 2, the control circuit board 40 may be provided with an electronic element, such as a processor, a memory, etc. The control circuit board 40 may include a first control circuit sub-board 41 and a second control circuit sub-board 42 that are fixed on different positions of the rack 10, such as the rack body 11. The first control circuit sub-board 41 is electrically connected to the second control circuit sub-board 42.

The first control circuit sub-board 41 may be electrically connected to the rack 10, such as the first drive component 12, so as to control the sliding position of the detection card transport base 20 relative to the rack 10.

The first control circuit sub-board 41 may be electrically connected to the abutting plate 50, such as the first circuit board 56. Therefore, the electromagnetic member 52 may apply the magnetic force to the pressing member 53, so that the pressing member 53 is adsorbed, thereby controlling the first heating member 54 and the second heating member 55 to heat the detection card.

The first control circuit sub-board 41 may be electrically connected to the transport assembly 60, such as the second driving assembly 6141, so as to control the third driving assembly 64 and the card holder 65 to slide together relative to the sliding frame 61.

The first control circuit sub-board 41 may be electrically connected to the transport assembly 60, such as the third driving assembly 64, so as to control the third driving assembly 64 to drive the card holder 65 to perform the centrifugal processing on the detection card.

The first control circuit sub-board 41 may be electrically connected to the second circuit board 84, so that the first control circuit sub-board 41 is indirectly connected to the detection chamber assembly 82, such as the heating element, so as to control the detection base 8211 to heat the detection card.

The first control circuit sub-board 41 may be electrically connected to the second circuit board 84, so that the first control circuit sub-board 41 is indirectly connected to the sampling chamber assembly 83, such as the heating element, so as to control the sampling chamber installation base 832 to heat the detection card.

The first control circuit sub-board 41 may be electrically connected to the detection assembly 90, such as the light generator 91, so as to control the light generator 91 to emit the excitation light.

The first control circuit sub-board 41 may be electrically connected to the detection assembly 90, such as the light receiver 92, so as to control the light receiver 92 to receive the fluorescence.

The second control circuit sub-board 42 may be installed above the rack 10, such as the rack body 11. In some embodiments, when a housing is disposed to the molecular diagnostic device 100, the second control circuit sub-board 42 may be disposed on a part of the housing corresponding to the rack body 11.

The second control circuit sub-board 42 may be electrically connected to the input device, such as the display, the keyboard, the scanning device 14, etc., so as to input the control instructions to the molecular diagnostic device 100, such as the control circuit board 40, through the input devices. It enables the molecular diagnostic device 100 to control the detection card transport base 20 and/or the detection card detection base 30 through the control circuit board 40.

In some embodiments, the second control circuit sub-board 42 and the first control circuit sub-board 41 may be integrated into one control circuit board.

The present disclosure provides a detection card, and the detection card may be applied to the molecular diagnostic device 100 in any one of the above embodiments, so as to complete the detection of samples loaded on the detection card and further process to form the diagnostic data.

As illustrated in FIG. 18, FIG. 18 is a structural schematic view of a detection card in some embodiments of the present disclosure. The detection card 93 is also known as a molecular diagnostic centrifugal detection card or a test card. The detection card 93 may include a body 94, an isolation layer 95, and a cover 96. The body 94 defines a sampling chamber, a flow channel, a waste liquid chamber, an isolation chamber, and a detection chamber. The isolation layer 95 covers a side of the body 94. The cover 96 covers the sampling chamber of the body 94.

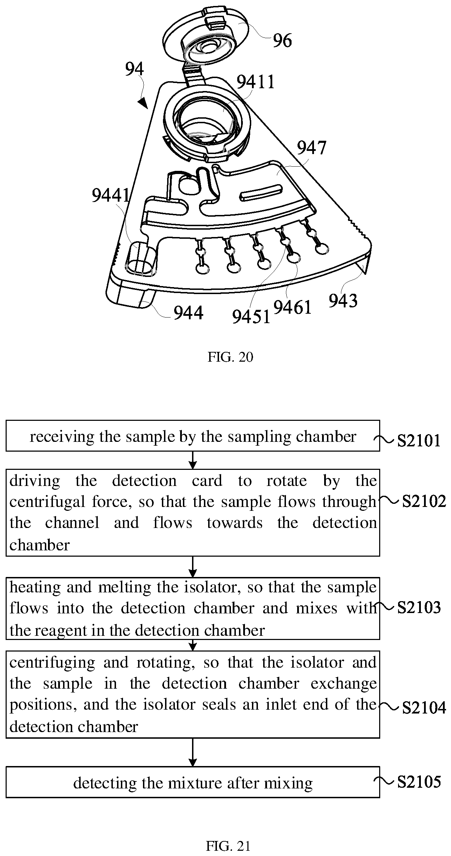

As illustrated in FIG. 18, FIG. 19, and FIG. 20, FIG. 19 is a cross-sectional structural schematic view of the detection card 93 of FIG. 18 along a line L-L, and FIG. 20 is a three-dimensional structural schematic view of the detection card 93 of FIG. 18. The body 94 is made of the rigid material, such as the plastic, etc.

The body 94 may have the plate-like structure as a whole. The body 94 is generally fan-shaped. In some embodiments, the body 94 may have a fan ring shape, a fan blade shape, or a pie shape. In some embodiments, the body 94 has a fan-shaped structure, and two straight sides and one curved side are connected end-to-end in sequence to form the fan-shaped structure. In some embodiments, the body 94 has the fan ring shape, and a straight side, an outer arc side, another straight side, and an inner arc side are connected end-to-end in sequence to form the fan ring shape. The body 94 may also have other shapes, which is not repeated here.

In some embodiments, an angle between the two straight sides of the fan-shaped structure of the body 94 may range from 40° to 60°, a diameter of the inner arc side may range from 10mm to 100mm, and a diameter of the outer arc side may range from 100mm to 200mm. At least six the detection card 93 having the fan-shaped structure with these sizes may be disposed on a detection plane of the molecular diagnostic device 100, forming a circular plane, so that at least six detection cards 93 may be detected simultaneously, thereby improving the overall detection efficiency and meeting large-scale detection needs.

The body 94 is provided with an installation part 941 that protrudes from a surface of the body 94 away from the isolation layer 95, and the installation part 941 is disposed on a side of the body 94 close to a center of the outer arc edge or close to the inner arc edge. A surface of the side of the body 94 close to the isolation layer 95 and corresponding to the installation part 941 is recessed to form the sampling chamber 9411, and the sampling chamber 9411 is configured for adding the sample. The sampling chamber 9411 is mainly configured for sample (liquid sample) pretreatment, and a pretreatment mode may include one or more, such as chemical treatment, heat treatment, enzyme treatment, and physical separation, etc. In some embodiments, a volume of the sampling chamber 9411 generally ranges from 200µL to 2000pL. A dry reagent may be disposed in the sampling chamber 9411 in advance. The reagent may be air dried or backed in situ, or the reagent may be added to the sampling chamber 9411 as a freeze-dried reagent.

A second clamping part 942 is disposed on the side of the body 94 away from the isolation layer 95, and the second clamping part 942 is also disposed on a part of the body 94 close to the outer arc side. The second clamping part 942 cooperates with the transport assembly 60, such as the first clamping part 654. In some embodiments, the second clamping part 942 may be a protrusion. It may be understood that the position of the first clamping part 654 may be exchanged with the position of the second clamping part 942. In addition, the first clamping part 654 and the second clamping part 942 may also be fixed together by other modes.

One end of one straight edge of the body 94 close to the outer arc is bent towards a direction away from the isolation layer 95, to form a first limiting part 943. One end of the other straight edge of the body 94 close to the outer arc protrudes towards the direction away from the isolation layer 95, to form a second limiting part 944. The first limiting part 943 and the second limiting part 944 of the body 94 cooperate with each other, so as to fix the detection card 93, so that the centrifugal processing of the detection card 93 is performed. A surface of the side of the body 94 close to the isolation layer 95 and corresponding to the second limiting part 944 is recessed, to form the waste liquid chamber 9441. Multiple plug-in groups are disposed on the side of the body 94 away from the isolation layer 95, and between the first limiting part 943 and the second limiting part 944. The plug-in groups are uniformly distributed along an extending direction of the outer arc side. Each plug-in group may include a first plug-in part 945 and a second plug-in part 946. The center of the outer arc side is located on a connection line between the first plug-in part 945 and the second plug-in part 946. The first plug-in part 945 is disposed between the installation part 941 and the second plug-in part 946. The setting of the first plug-in part 945 and the second plug-in part 946 may coordinate with the detection card detection base 30, such as the isolation groove 8213 and the detection groove 8212.

A surface of a side of the body 94 close to the isolation layer 95 and corresponding to the first plug-in part 945 is recessed, to form the isolation chamber 9451. The isolation chamber 9451 is provided with a meltable isolator. The isolator may switch between a molten state and an unmelted state (usually solid). When there is no the detection card 93 for detection, the isolator may be controlled to be in the unmelted state. In this case, the isolator may prevent the sample from entering the detection chamber 9461 through the flow channel (as illustrated in FIG. 20). In some embodiments, the isolator may be a paraffin wax, a microcrystalline wax, a synthetic wax, or a natural wax, etc.

A surface of the side of the body 94 close to the isolation layer 95 and corresponding to the second plug-in part 946 is recessed, to form the detection chamber 9461. The detection chamber 9461 is provided with a reagent. A side of the isolation chamber 9451 close to the isolation layer 95 is communicated with a side of the detection chamber 9461 close to the isolation layer 95. The isolator in the isolation chamber 9451 may also be configured to seal an isolate the reagent, so as to prevent the reagent entering the isolation chamber 9451 in reverse. The reagent is maintained in the detection chamber 9461. In the process of detection, the isolator may be controlled to be in the molten state. In this case, the sample may enter the detection chamber 9461 through the sampling chamber 9411, so that the sample reacts with the reagent in the detection chamber 9461, completing the detection.

The reagent and the meltable isolator may be stacked with each other and disposed in the detection chamber 9461. In some embodiments, the isolator may be the paraffin wax, the microcrystalline wax, the synthetic wax, or the natural wax, etc. A characteristic of the isolator is that the isolator is solid at a room temperature and a low temperature, and the isolator becomes liquid after the isolator is heated to a specific temperature. The isolator has no inhibitory effect on a nucleic acid amplification reaction. In some embodiments, the reagent may be the dry reagent. The dry reagent includes a primer used in the amplification reaction, and one or more of a deoxyribonucleic acid (DNA) binding dye, an enzyme, magnesium sulfate, potassium chloride, and nucleoside triphosphate (dNTPs). The dry reagent is disposed into the detection chamber 9461 in a liquid form, and the dry reagent is formed through a drying process. A temperature of the drying process is lower than a melting temperature of the isolator. The drying process includes air drying, heating drying, or freeze-drying, etc. In the process of detecting and heating, both the reagent and the isolator are in the liquid form. Because a specific gravity of the isolator is less than that of the reagent, the isolator may be separated from the detection chamber 9461 under the action of the centrifugal field. Therefore, the isolator does not affect the reaction and detection.

In some embodiments, the isolator in the molten state is disposed in the detection chamber 9461, and the isolator is solidified by natural solidification or cooling solidification. When there is no detection, the isolator may be controlled to be in the unmelted state, in this case, the reagent may be sealed, isolated, and stored through the isolator. When the detection needs to be performed, the isolator may be controlled to be in the molten state, for example, the detection card 93 is heated to melt the isolator. In this case, the isolator may flow out of the detection chamber 9461 and flow into the isolation chamber 9451 under the action of the centrifugal force. The sample may enter the detection chamber 9461. Then, the isolator is solidified again, so as to seal an opening part of the detection chamber 9461, thereby forming mutual isolation and sealing for multiple detection chambers 9461, so that reaction or detection are independently performed in the multiple detection chambers 9461.

The side of the body 94 close to the isolation layer 95 defines a flow channel 947, so that the sampling chamber 9411 is communicated with the isolation chamber 9451.

A surface of the side of the body 94 away from the isolation layer 95 and corresponding to the flow channel 947 defines a contact groove 948, so that the contact groove 948 cooperates with the contact part 8324. For example, the contact part 8324 may be disposed in the contact groove 948, so that the flow channel 947 is heated, avoiding condensation of the water vapor in the flow channel 947, thereby reducing the impact on subsequent detection processes and improving detection accuracy. In some embodiments, the contact groove 948 may be omitted.

The isolation layer 95 may have a film-like structure, or other structures. The isolation layer 95 may be made of a material, such as a pressure-sensitive adhesive, a UV curable adhesive, or an optical grade double-sided adhesive. The isolation layer 95 and the body 94 may have similar materials. The isolation layer 95 may be attached on the body 94. The isolation layer 95 may be sealed and fixed on the body 94 by a mode, such as ultrasonic welding, laser welding, or adhesive sealing, etc. Therefore, the flow channel 947, the waste liquid chamber 9441, the isolation chamber 9451, and the detection chamber 9461 are isolated. The isolation layer 95 may be integrated with the body 94.

The cover 96 covers the opening part of the sampling chamber 9411, so as to seal and isolate the sampling chamber 9411. When the sample needs to be added, the cover 96 may be opened, the sample may be added to the sampling chamber 9411, and then the cover 96 may be closed. The cover 96 may block water and allow air to pass through. That is, the cover 96 may discharge the water vapor generated during heating, reduce the air pressure in the molecular diagnostic centrifugal detection card, and ensure good air permeability. It may also block the escape of pollutants, such as aerosols and biomolecules generated during the amplification reaction, avoiding pollution to person and environment during detection.

When the detection card 93 rotates centrifugally, the sample liquid extends or passes through the sampling chamber 9411 and the flow channel 947. In this case, the isolator is solid, and the solid isolator may seal the detection chamber 9461 and prevent the sample liquid from flowing into the detection chamber 9461. After the sample liquid is heated, the isolator is melted and flows towards the detection chamber 9461, allowing communication between the flow channel 947 and the detection chamber 9461, and the sample liquid may flow into the detection chamber 9461. Because the specific gravity of the isolator is less than that of the reagent, the isolator may be separated from the reagent under the action of the centrifugal field. Therefore, the reaction and detection are not affected, and the detection chamber 9461 may be sealed.

It may be understood that when centrifuging the detection card 93 filled with the samples, in order to ensure that the detection card 93 filled with the samples is uniformly distributed on the card holder 65 along the circumferential direction and reduce the noise caused by uneven distribution of the detection card 93 in the process of the centrifugation, the detection card 93 without the samples may be used as a balancing card. The detection card 93 without the samples and the detection card 93 filled with the samples are uniformly distributed on the card holder 65 along the circumferential direction, and the centrifugation is performed.

In some embodiments, when the detection card 93 is used as the balancing card, the isolation chamber 9451 may not be provided with the isolator.

In some embodiments, when the detection card 93 is used as the balancing card, the reagent and the isolator may not be disposed in the detection chamber 9461.

In some embodiments, when the detection card 93 is used as the balancing card, the body 94 may be integrated with the cover 96.

In some embodiments, when the detection card 93 is used as the balancing card, the sampling chamber 9411 may not be provided with the reagent.

In some embodiments, when the detection card 93 is used as the balancing card, at least one of the flow channel 947, the isolation chamber 9451, the waste liquid chamber 9441, and the cover 96 may be omitted.

In some embodiments, when the detection card 93 is used as the balancing card, the sampling chamber 9411 may be omitted. A third clamping part integrated with the body 94 is disposed at a position where the sampling chamber 9411 is disposed. In some embodiments, the third clamping part is a bump and installed on the sampling chamber installation base 832.

The balancing card may play a role in balancing and reducing noise during centrifugal treatment, and a reference membrane may be disposed on the bottom of the detection chamber 9461 to replace the reagent and the isolator, thereby correcting the detection assembly 90 of the molecular diagnostic device 100 and improving the detection accuracy of the detection assembly 90 of the molecular diagnostic device 100.

In some embodiments, a composition of the reference membrane may include a fluorescent indicator. In some embodiments, the components of the reference membrane may also include an auxiliary reagent, and the auxiliary reagent may help the fluorescent indicator form a film-like structure. The fluorescent indicator refers to a type of substance that may generate the fluorescence through the laser irradiation. The fluorescent indicator may generate the fluorescence when excited by the excitation light of the light generator 91, and the light receiver 92 may receive the fluorescence generated by the fluorescent indicator. When the balancing card is placed in different detection chamber assemblies 82 and different sampling chamber assemblies 83, the detection assembly 90 of the detection card detection base 30 is detected, so as to ensure the normal and accurate operation of the detection assembly 90. Therefore, the balancing card has the function of correcting the detection assembly 90 of the molecular diagnostic device 100, thereby improving the detection accuracy of the molecular diagnostic device 100.

In some embodiments, the reference membrane may be replaced by a liquid fluorescent indicator and other types of auxiliary reagents, so as to fill the detection chamber 9461. It may be understood that the form of the fluorescent indicator is not limited, as long as the fluorescent indicator may generate the fluorescence when excited by the excitation light of the light generator 91.

Some embodiments of the present disclosure further provide a method for detection based on the detection card 93 described above. The method may be performed by the molecular diagnostic device 100 in any one of the above embodiments. As illustrated in FIG. 21, FIG. 21 is a schematic view illustrating a usage process of the detection card of FIG. 20. The method includes the following operations.

At block S2101, the method may include receiving the sample by the sampling chamber.

After the sample is added to the sampling chamber 9411, it is necessary to perform heating pretreatment on the sample. The heating modes may include metal heating block, heated airflow, electromagnetic wave (infrared radiation, laser, microwave), etc. A heating area of the detection card 93 may be an area near the sampling chamber. In the process of heating, a temperature is increased to a specified temperature, such as 90°C, by heating; after reaching the specified temperature, maintaining the specified temperature for 3 minutes to 10 minutes according to the specified requirements, so as to achieve pre-treatment; and after the preprocessing is completed, the temperature of the sample liquid is reduced to another specified temperature, such as 60°C.

At block S2102, the method may include driving the detection card to rotate by the centrifugal force, so that the sample flows through the channel and flows towards the detection chamber.

The rotation of the detection card is controlled, so as to perform centrifuging. For example, a rotation direction may be controlled to be clockwise, a rotation speed is greater than 1000rpm, and a rotation time ranges from about 10 seconds to about 15 seconds. By rotating, the sample liquid may flow from the sampling chamber to the detection chamber through the flow channel, facilitating the subsequent filling of the sample in the detection chamber. The excess sample liquid may enter the waste liquid chamber.

At block S2103, the method may include heating and melting the isolator, so that the sample flows into the detection chamber and mixes with the reagent in the detection chamber.

It is necessary to heat the area near the detection chamber, so as to make the temperature higher than the melting point of the isolator, thereby melting the isolator and mixing the sample with the reagent in the detection chamber.

At block S2104, the method may include centrifuging and rotating, so that the isolator and the sample in the detection chamber exchange positions, and the isolator seals an inlet end of the detection chamber.

The rotation and control of the detection card is performed again. The motor may rotate clockwise, and a rotation speed is greater than 1000rpm, and a rotation time ranges from 10 seconds to 15 seconds. In this case, due to the rotation of the detection card, the sample enters the detection chamber, and the isolator and the aqueous solution in the detection chamber exchange positions. The isolator is transferred to the inlet end of the detection chamber, thus completing the sealing of each detection chamber. Then the control parameters of the motor are changed, so that the detection card alternately rotates clockwise and counterclockwise. For example, the detection card may be controlled to rotate clockwise, the rotation speed is 3000rpm, and the rotation time is 1 second; and then the detection card may be controlled to rotate counterclockwise, the rotation speed is 3000rpm, and the rotation time is 1 second; and the detection card alternately rotates 10 times to 15 times. By alternately rotating clockwise and counterclockwise, the sample and reagent in the detection chamber may be completely dissolved and mixed.

At the same time, the isolator is heated and melted, and flows to the inlet end of the detection chamber under the action of centrifugal force, so as to seal the inlet end of the detection chamber.

At block S2105, the method may include detecting the mixture after mixing.

In this operation, the amplification reaction and the detection are performed. When real-time detection is performed, the detection and the amplification reaction are performed simultaneously. When the endpoint detection is performed, the detection is performed after amplification is complete. The amplification reaction may be performed by the following mode: heating the vicinity of the detection chamber and controlling the temperature to range from 60°C to 75°C; and after reaching the specified temperature, remaining the specified temperature for 30 minutes to 60 minutes according to the specified requirements, so as to complete the amplification reaction.

The present disclosure further describes the operation process of detecting the sample through using the molecular diagnostic device 100 and the detection card 93. The operation process is as follows.

The control circuit board 40 sets basic information, such as sample quantity, type, heating temperature, rotation speed, and operating parameters for the detection card 93 through the input device. After completing the basic information settings, the detection of the detection card 93 may be further started through the input device.

The detection card 93 with the sample is loaded.

Under the control of the control circuit board 40, the first driving assembly 12 controls the lead screw 13 to rotate, so that the abutting plate 50 drives the transport assembly 60 to slide on the first guiding rail 111 along a direction away from the detection card detection base 30. The abutting plate 50 reaches the predetermined position, and the first driving assembly 12 stops running.

Under the control of the control circuit board 40, the second driving assembly 6141 drives the transport member 62 to slide on the second guiding rail 613 along a direction close to the scanning device 14 through the lead screw 6142. The transport member 62 reaches the predetermined position, and the second driving assembly 6141 stops running.

The detection card 93 loaded with the sample is placed on the transport member 62, such as disposed in the concave part 653 of the card holder 65. The detection card 93, such as the installation part 941, is disposed in the receiving hole 655. The detection card 93, such as the second clamping part 942, is fixed to the first clamping part 654 of the card holder 65, completing the placement of one detection card 93.

The control circuit board 40 scans the information identifier of the detection card 93 on the card holder 65 through the scanning device 14, and determines whether the detection card 93 is a user's predetermined detection card 93. If not, a warning prompt may be issued. If so, proceed to the next operation.

Under the control of the control circuit board 40, the third driving assembly 64 begins to rotate and drives the card holder 65 to rotate, so that the concave part 653 where the next detection card 93 may be placed, reaches the predetermined position, and then the placement of the next detection card 93 is operated.

After placing the detection cards 93 in the concave part 653 of the card holder 65, the detection cards 93 may be uniformly distributed on the card holder 65 along the circumferential direction. When the number of the detection cards 93 is insufficient, so that the detection cards 93 are not uniformly distributed on the card holder 65 along the circumferential direction, the balancing card may be placed in the concave part 653 of the card holder 65, so as to ensure that the balancing card and the detection card 93 are uniformly distributed on the card holder 65 along the circumferential direction. At this point, the placement of the detection card 93 is completed.

Under the control of the control circuit board 40, the second driving assembly 6141 drives the transport member 62 to slide on the second guiding rail 613 along the direction away from the scanning device 14 through the lead screw 6142. The transport member 62 reaches the predetermined position, and the second driving assembly 6141 stops running.

Under the control of the control circuit board 40, the first driving assembly 12 controls the lead screw 13 to rotate, so that the abutting plate 50 drives the transport assembly 60 to slide on the first guiding rail 111 along the direction close to the detection card detection base 30. When the transport member 62, such as the card holder 65, is about to touch the support base 80, such as the support base body 81, the first positioning member 656 that is disposed on the side of the card holder 65 facing the support base body 81 may extend into the second positioning member 813. Due to the special structure of the first positioning member 656, the first positioning member 656 is easily placed inside the second positioning member 813, achieving the positioning of the detection card 93.

When the card holder 65 is in direct contact with the support base 80, such as the support base body 81, the detection card 93 may be located on the support base 80. The detection card 93, such as the installation part 941, may be placed in the sampling chamber installation groove 8322 of the sampling chamber installation base 832. The detection card 93, such as the first plug-in part 945, may be placed in the optical detection assembly 821, such as the isolation groove 8213. The detection card 93, such as the second plug-in part 946, may be placed in the light detection member 821, such as the detection groove 8212. The detection card 93, such as the first limit part 943, may be placed in the support base body 81, such as the extending groove 814, and may be in direct contact with the assembly table 815. The detection card 93, such as the second limit part 944, may be placed in the support base body 81, such as the extending groove 814, and may be in direct contact with the assembly table 815. At this point, the placement of the detection cards 93 on the support base 80 is completed.

The abutting plate 50 reaches the predetermined position, and the first driving assembly 12 stops running. In this case, the abutting plate 50, such as the first heating member 54, is in direct contact with the part of the detection card 93 where the isolation chamber 9451 and the detection chamber 9461 are formed. The pressing element 53 clamps and fixes the cover 96, and the fixing of the pressing element 53 is released.

Under the control of the control circuit board 40, the heating element in the sampling chamber assembly 83, such as the heating element in the sampling chamber installation base 832, and the heating element in the second heating member 55 are heated, so as to heat the detection card 93, such as the sampling chamber 9411. The heating preprocessing of the sample is implemented.

Under the condition that there is the electromagnetic member 52, it is necessary to eliminate the electromagnetic force under the control of the control circuit board 40, so that the pressing member 53 is disposed above the card holder 65 and fixed with the card holder 65. In addition, the card holder 65 more closely presses against the pressing member 53 through the force that is generated by the electromagnet, the permanent magnet, etc. disposed in the card holder 65.

Under the control of the control circuit board 40, the first driving assembly 12 controls the lead screw 13 to rotate, so that the abutting plate 50 drives the transport assembly 60 to slide on the first guiding rail 111 along a direction away from the detection card detection base 30. The abutting plate 50 reaches the predetermined position, and the first driving assembly 12 stops running.

Under the control of the control circuit board 40, the third driving assembly 64 begins to rotate, and drives the card holder 65 to rotate, achieving the centrifugal processing of the detection card 93.

Under the control of the control circuit board 40, the detection card 93 is transported again to the support base 80, such as the support base body 81.

Under the control of the control circuit board 40, the heating element in the first heating member 54 is controlled to heat, and the heating element in the light detection element 821 is controlled to heat. Secondary heating of the detection card 93 is implemented.

Then, the centrifugal processing of the detection card 93 is performed again.

Finally, the detection card 93 is transported to the support base 80, such as the support base body 81.

Under the control of the control circuit board 40, the light generator 91 emits the excitation light, so as to excite the mixture in the detection chamber 9461 of the detection card 93 in the detection groove 8212. The light receiver 92 receives the fluorescence. The control circuit board 40 receives and processes the detection signal to form the diagnostic data.

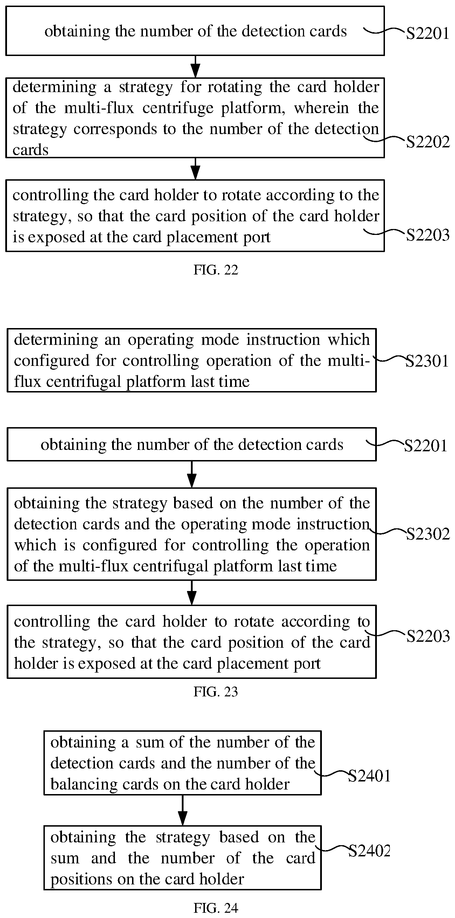

The present disclosure further provides a control method based on a multi-flux centrifugal platform. The control method may be applied to the molecular diagnostic device 100 mentioned above and may detect the detection card 93 mentioned above. The multi-flux centrifugal platform may be the molecular diagnostic device 100 mentioned above. The method may include a detection card placement process and a detection card measurement process. In the detection card placement process, the detection card 93 is placed on the multi-flux centrifugal platform, such as the molecular diagnostic device 100. As illustrated in FIG. 22, FIG. 22 is a flowchart of the control method based on the multi-flux centrifugal platform in some embodiments of the present disclosure. The control method may include the following operations.

At block S2201, the control method may include obtaining the number of the detection cards.

When the detection card 93 is detected through the multi-flux centrifugal platform, such as the molecular diagnostic device 100, the centrifugation treatment of the detection card 93 needs to be performed. The multi-flux centrifugal platform, such as the molecular diagnostic device 100, may be provided with the card holder 65 configured for supporting the detection card 93. Multiple card positions (i.e. the concave part 653 in FIG. 9) may be disposed or defined on each card holder 65. For example, in FIG. 9, there are six card positions disposed or defined on the card holder 65. It may be understood that different multi-flux centrifugal platforms have different numbers of the card positions on the card holder.

When centrifuging the detection card 93 through the multi-flux centrifugal platform, such as the molecular diagnostic device 100, the detection cards 93 may be symmetrically placed on the card positions of the card holder 65, so that a balanced weight distribution on the card holder 65 is achieved when the detection cards 93 are placed on the card positions. When placement of the detection cards 93 on the card positions causes uneven weight distribution on the card holder 65, an eccentric force may be generated, so that the multi-flux centrifugal platform, such as the molecular diagnostic device 100, generates a large amount of noise during centrifugation.

In the conventional measurement process, in the multi-flux centrifugal platform, such as the molecular diagnostic device 100, the card holder 65 is always filled with the maximum number of detection cards 93, thereby avoiding the problem of uneven weight distribution on the card holder 65. However, there may also be a special situation where the number of the detection cards 93 is insufficient to fill the card holder 65 during the use of the multi-flux centrifugal platform, such as the molecular diagnostic device 100, resulting in uneven weight distribution on the card holder 65. The situation is particularly prone to occur when manually placing the detection card 93.

For the multi-flux centrifugal platform, such as the molecular diagnostic device 100, when the card holder 93 slides towards the scanning device 14, only one card position may be exposed at a card placement port. Then, after rotating, other card positions are exposed at the card placement port. When the detection cards 93 are manually placed on the card holder 65, which further increases the difficulty of placing the detection cards 93, making it extremely difficult to achieve the balanced weight distribution on the card holder 65. The control method provided by the present disclosure may assist in manually placing the detection cards 93, thereby reducing the error rate of manually placing the detection cards 93 and solving the problem that the multi-flux centrifugal platform, such as the molecular diagnostic device 100, generates a large amount of noise during centrifugation.

In some embodiments, when placing the detection cards 93 on the card holder 65, the number of detection cards 93 is particularly important as the basic data. In some embodiments, the number of detection cards 93 may be inputted by the user into the system of the multi-flux centrifugal platform, such as the molecular diagnostic device 100, through the input device, such as the display, the keyboard, and the scanning device 14 according to the actual situation. Therefore, the system of the multi-flux centrifugal platform, such as the molecular diagnostic device 100, may assist the user in completing the balancing process of the detection cards 93 on the card holder 65 based on the number of detection cards 93.

In some embodiments, as shown in FIG. 9, there are six card positions on the card holder 65. When the number of the detection cards is one or five, it is not possible to achieve the weight distribution balance on the card holder 65. Therefore, when the number of the detection cards 93 is insufficient to achieve the weight distribution balance on the card holder 65, the balancing card described in any one of the above embodiments may be introduced, so that the number of cards, such as the detection cards 93 and the balancing cards, is sufficient to achieve the weight distribution balance on the card holder 65. For example, when the number of the detection cards is one, one balancing card is supplemented, and a sum of the number of the cards is two, which is sufficient to achieve the weight distribution balance on the card holder 65. For example, when the number of the detection cards is five, one balancing card is supplemented, the sum of the number of the cards is six, which is sufficient to achieve the weight distribution balance on the card holder 65.

At block S2202, the control method may include determining a strategy for rotating the card holder of the multi-flux centrifugal platform, wherein the strategy corresponds to the number of the detection cards.

For the multi-flux centrifugal platform, such as the molecular diagnostic device 100, when the card holder 93 slides towards the scanning device 14, only one card position may be exposed at the card placement port. Then, after rotating, other card positions are exposed at the card placement port. Therefore, the detection card 93 may achieve the weight distribution balance on the card holder 65, or the detection card 93 and the balancing card may achieve the weight distribution balance on the card holder 65. The card position may be exposed at the card placement port, so as to guide the user to place the detection card 93. Furthermore, the multi-flux centrifugal platform, such as the molecular diagnostic device 100, assists the user in placing the detection cards 93 on the card holder 65. Therefore, the method may assist in manually placing the detection card 93, thereby reducing the error rate of manually placing the detection cards 93 and solving the problem that the multi-flux centrifugal platform, such as the molecular diagnostic device 100, generates a large amount of noise during centrifugation.

In some embodiments, when the number of the detection cards 93 is determined, the multi-flux centrifugal platform, such as the molecular diagnostic device 100, may determine the required number and position of the card positions based on the number of detection cards 93. The card holder rotation strategy of how the card holder 65 rotates at the card placement port is determined based on the required number and position of the card positions, so that the card holder 65 rotates according to the strategy. The user is prompted to place the detection card 93 or the balancing card for balancing through exposing the card positions at the card placement port.

In some embodiments, in order to achieve the weight distribution balance on the card holder 65, a preset algorithm is configured to perform balance calculation based on the number of the detection cards 93 and the number of the card positions on the card holder 65, so that the strategy is obtained. In some embodiments, the preset algorithm may be set in the system of the multi-flux centrifugal platform, such as the molecular diagnostic device 100. In some embodiments, the strategy pre-corresponds to the number of the detection cards 93, to form a relationship pre-correspondence. The relationship pre-correspondence is pre-set in the multi-flux centrifugal platform, such as the molecular diagnostic device 100 system. Therefore, when the number of the detection cards 93 is obtained, the strategy can be obtained by searching in the relationship pre-correspondence. In some embodiments, the strategy may include rotating the card holder 65, so that the required card positions are sequentially exposed. Therefore, the cards, such as the detection cards 93 and the balancing cards, are placed on the exposed card positions at the card placement port. The strategy may further include a rotation direction of the card holder 65, such as clockwise rotation or counterclockwise rotation. The strategy may further include reminding the user to place the detection card 93 or the balancing card on the card position through an indicator light or a voice message, etc. when the card position is exposed at the card placement port. In some embodiments, when the detection card 93 is placed at the card placement port, the user is reminded to place the detection card 93 through voice broadcasting. In some embodiments, when the balancing card 93 is placed at the card placement port, the indicator light may flash to remind the user to place the balancing card 93.

At block S2202, the control method may include controlling the card holder to rotate according to the strategy, so that the card position of the card holder is exposed at the card placement port.

After the strategy is formed, the rotation of the card holder 65 may be controlled according to the strategy, so that the required card positions are exposed sequentially and one by one at the card placement port. The user only needs to place the detection card 93 or the balancing card when the card position is exposed at the card placement port, thereby completing the balance, and ensuring the safe operation of the multi-flux centrifugal platform, such as the molecular diagnostic device 100.

In some embodiments, as illustrated in FIG. 23, FIG. 23 is a flowchart of a control method based on the multi-flux centrifugal platform in some embodiments of the present disclosure. Before the operation S2202, the method further includes the following operation.

At block S2301, the method may include determining an operating mode instruction which configured for controlling operation of the multi-flux centrifugal platform last time.

The operating mode instruction for the multi-flux centrifugal platform, such as the molecular diagnostic device 100, may include a mode instruction that requires the balancing card for balancing, and a mode instruction that does not require the balancing card for balancing. Each of the two operating mode instructions has its own characteristics. For example, the mode instruction that requires the balancing card for balancing may generate a strategy that requires participation of the balancing card when the detection card 93 is used for balancing, and the balancing card may not be removed when detection of the detection card 93 is completed. For example, during balancing by using the detection card 93, the mode instruction that does not require the balancing card for balancing, may generate the strategy preferentially based on the detection card 93. When the detection card 93 cannot complete the balancing, the strategy that requires participation of the balancing card is generated. Under the condition that the balancing card is used or participates, it needs to remove the balancing card when detection of the detection card 93 is completed.

The setting of two operating mode instructions may enable the user to choose according to needs, so as to better improve detection efficiency. In some embodiments, the operating mode instructions of the multi-flux centrifugal platform, such as the molecular diagnostic device 100, may be input through the input device, such as the display, the keyboard, and the scanning device 14, so that the multi-flux centrifugal platform, such as the molecular diagnostic device 100, is controlled. It may be understood that the operating mode instructions of the multi-flux centrifugal platform, such as the molecular diagnostic device 100, may also include other operating mode instructions, which is not repeated here.

The operating mode instruction which is configured for controlling the operation of the multi-flux centrifugal platform last time may make the user consider whether there is the balancing card on the card holder 65 when the current detection card 93 is detected, whether the balancing card needs to participate when the current detection card 93 is used for balancing, and whether the balancing card needs to be removed or placed, etc.

The operation S2202 may include the following operation.

At block S2302, the method may include obtaining the strategy based on the number of the detection cards and the operating mode instruction which is configured for controlling the operation of the multi-flux centrifugal platform last time.

The operating mode instruction which is configured for controlling the operation of the multi-flux centrifugal platform last time may allow the balancing card to be left on the card holder 65. Therefore, when the strategy is generated, it is necessary to consider whether there is the balancing card on the card holder 65 based on the operating mode instruction which is configured for controlling the operation of the multi-flux centrifugal platform last time. In some embodiments, when the operating mode instruction which is configured for controlling the operation of the multi-flux centrifugal platform last time, is the mode instruction that requires the balancing card for balancing, there is the balancing card on the current card holder 65. In some embodiments, when the operating mode instruction which is configured for controlling the operation of the multi-flux centrifugal platform last time, is the mode instruction that does not require the balancing card for balancing, there is no the balancing card on the current card holder 65. In some embodiments, when there is the balancing card in the current card holder 65, the balancing card may be removed first, and then a current operating mode instruction controlling the operation of the multi-flux centrifugal platform may be executed. That is, the strategy may be generated according to the number of detection cards 93. In some embodiments, when there is the balancing card on the current card holder 65, the strategy that requires the participation of the balancing card may be directly generated, so as to reduce the operation of removing the balancing card, thereby improving detection efficiency. In some embodiments, when there is the balancing card on the current card holder 65 and the strategy that requires the participation of the balancing card cannot be directly generated, the balancing card may be removed, and the strategy that does not require the participation of the balancing card may be generated. Therefore, the intelligence of the multi-flux centrifugal platform, such as the molecular diagnostic device 100, may be improved.

It may be understood that the operation S2301 may be performed simultaneously with the operation S2201, or the operation S2301 is performed first and then the operation S2201 is performed, or the operation S2201 is performed first and then the operation S2301 is performed.

In some embodiments, as illustrated in FIG. 24, FIG. 24 is a flowchart of a control method based on the multi-flux centrifugal platform in some embodiments of the present disclosure. The operation S2301 may include: determining the operating mode instruction which is configured for controlling the operation of the multi-flux centrifugal platform last time as the mode instruction that requires the balancing card for the balancing.

At block S2302, the method may include the following operations.

At block S2401, the method may include obtaining sum of the number of the detection cards and the number of the balancing cards on the card holder.

When the operating mode instruction which is configured for controlling the operation of the multi-flux centrifugal platform last time, is the mode instruction that required the balancing card for balancing, there is the balancing card placed on the current card holder 65. Therefore, before executing the current operating mode instruction controlling the operation of the multi-flux centrifugal platform, the balancing card is removed. The current operating mode instruction controlling the operation of the multi-flux centrifugal platform requires the balancing card, so that the balancing card is placed again, resulting in cumbersome operation steps, thereby reducing the detection efficiency. In order to improve the detection efficiency, when the detection may be completed without removing the balancing card, the balancing card is not removed.

Therefore, it is necessary to determine whether the strategy that requires the participation of the balancing card may be generated, which may be determined through the sum of the number of the detecting cards 93 and the number of the balancing cards on the card holder 65.

At block S2402, the method may include obtaining the strategy based on the number of the card positions on the card holder and the sum of the number of the detecting cards and the number of the balancing cards on the card holder.

When there is the balancing card, corresponding strategy may be obtained based on the sum of the number of the detecting cards 93 and the number of the balancing cards on the card holder 65. The strategy may be the strategy that requires the participation of the balancing card, or the strategy that does not require the participation of the balancing card. In some embodiments, in response to a result that the balancing cannot be performed based on the number of the card positions on the card holder 65 and the sum of the number of the detecting cards 93 and the number of the balancing cards on the card holder 65, the balancing is directly performed based on the number of the detection cards 93, so that the strategy is obtained. In some embodiments, in response to the result that the balancing cannot be performed based on the number of the card positions on the card holder 65 and the sum of the number of the detecting cards 93 and the number of the balancing cards on the card holder 65, the card position where the balancing card is placed is exposed at the card placement port, so that the user may remove the balancing card and execute the strategy to place the detection card 93 on the card holder 65. In some embodiments, in response to the result that the balancing cannot be performed based on the number of the card positions on the card holder 65 and the sum of the number of the detecting cards 93 and the number of the balancing cards on the card holder 65, the current operating mode instruction controlling the operation of the multi-flux centrifugal platform, is recorded as the mode instruction that does not require the balancing card for balancing. It is impossible to balance, therefore, the balancing card must be removed. When the current operating mode instruction controlling the operation of the multi-flux centrifugal platform, is the mode instruction that requires the balancing card for balancing, there is no balancing card after the detection is completed, it may cause a system error, further resulting in the inability to next detection card balancing. Therefore, the current operating mode instruction controlling the operation of the multi-flux centrifugal platform, may be changed to the mode instruction that does not require the balancing card for balancing, so as to facilitate the smooth performance of the next detection card balancing. In some embodiments, when the detection is completed, one card position is exposed at the card placement port, so that the user may place the balancing card on the card position. Therefore, the current operating mode instruction controlling the operation of the multi-flux centrifugal platform is still the mode instruction that requires the balancing card for balancing, and the next detection card balancing may be smoothly performed.

In some embodiments, the operation S2402 may include: the strategy is obtained by performing the balancing based on the number of the card positions on the card holder 65 and the sum of the number of the detecting cards 93 and the number of the balancing cards on the card holder 65, in response to a result that the balancing is able to be performed based on the number of the card positions on the card holder 65 and the sum of the number of the detecting cards 93 and the number of the balancing cards on the card holder 65.

At block S2203, the method may include: controlling the card holder to rotate according to the strategy in response to an initial position of the card position corresponding to the balancing card not being at the card placement port, so that the card position corresponding to the balancing card skips the card placement port, and an empty card position of the card holder is exposed at the card placement port.

For the multi-flux centrifugal platform, such as the molecular diagnostic device 100, when the card holder 93 slides towards the scanning device 14, only one card position may be exposed at the card placement port. In some embodiments, this card position may be the card position with the balancing card, the card holder 65 is rotated according to the strategy, so as to expose other required card position, so that the detection card 93 is placed on the card holder 65. In some embodiments, this card position is the empty card position, which indicates that the balancing card is placed in another position. The card holder 65 is rotated according to the strategy, because there is the balancing card placed on the required card position, and the balancing card does not need to be removed. Therefore, the card position corresponding to the balancing card may be skipped at the card placement port, so as to expose other required card positions, thereby improving the detection efficiency and preventing the user from taking out the detection card 93 incorrectly.

In some embodiments, the operation S2301 may include determining the operating mode instruction which is configured for controlling the operation of the multi-flux centrifugal platform last time as the mode instruction that does not require the balancing card for balancing.

The operation S2302 may include: obtaining the strategy based on the number of the detection cards 93 and the number of the card positions of the card holder 65.

It may be understood that when the operating mode instruction which is configured for controlling the operation of the multi-flux centrifugal platform last time is the mode instruction that does not require the balancing card for balancing, there is no the balancing card on the current card holder 65. Furthermore, when executing the current operating mode instruction controlling the operation of the multi-flux centrifugal platform, the strategy may be obtained by balancing only based on the number of the detection cards and the number of the card positions of the card holder 65. In some embodiments, the obtained strategy may be the strategy that does not require the participation of the balancing cards, or the strategy that requires the participation of the balancing cards. That is, in response to the result that the balancing cannot be performed based on the number of the detection cards 93 and the number of the card positions of the card holder 65, the balancing is performed by using the balancing cards, so that the strategy is obtained.

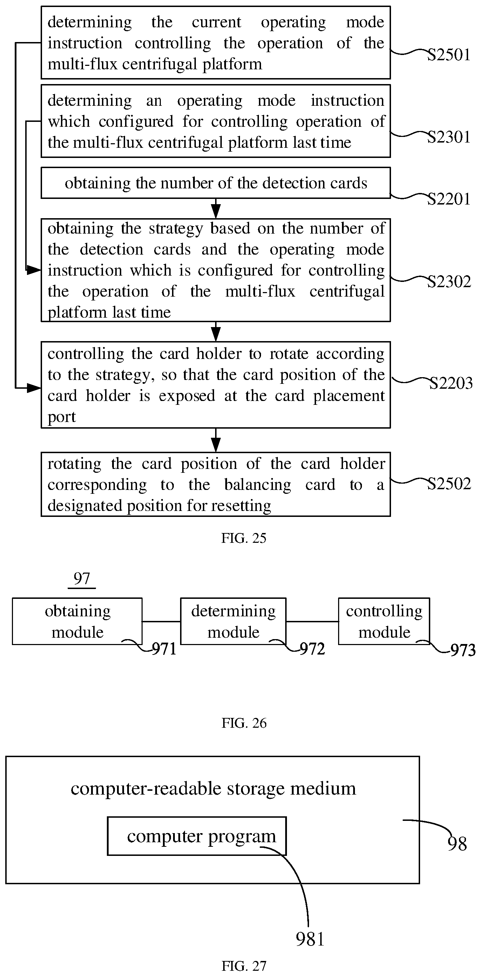

In some embodiments, as illustrated in FIG. 25, FIG. 25 is a flowchart of a control method based on the multi-flux centrifugal platform in some embodiments of the present disclosure. Before the operation S2203, the method may further include the following operation.

At block S2501, the method may include determining the current operating mode instruction controlling the operation of the multi-flux centrifugal platform as the mode instruction that requires the balancing card for balancing.

After the operation S2203, the method further includes the following operation.

At block S2502, the method may include rotating the card position of the card holder 65 corresponding to the balancing card to a designated position for resetting.

When the current operating mode instruction controlling the operation of the multi-flux centrifugal platform is the mode instruction that requires the balancing card for balancing, after the detection card 93 is completed, the balancing card needs to be retained on the card holder 65. Therefore, it is necessary to reset the balancing card, so as to facilitate the smooth performance of the next detection card balancing operation and improve the detection efficiency. The designated position may be the card position that is exposed at the card placement port when the card holder 93 slides towards the scanning device 14. In some embodiments, considering the balancing effect, the designated position may also be a card position opposite to the card position that is exposed at the card placement port when the card holder 93 slides towards the scanning device 14. Of course, the designated position may be other card positions, for example, the card position without the balancing card in the card holder 65 is rotated to a position corresponding to the card placement port.

It may be understood that the operation S2501 may or may not be the same operation as the operation S2301.

In some embodiments, when the card position is exposed at the card placement port, according to the strategy, the user may be reminded to place the detection card 93 or the balancing card on the card position through the indicator light or the voice, etc. Therefore, the user may be reminded to remove or take out the detection card 93 or the balancing card. In some embodiments, when the card, such as the detection card 93 or the balancing card, is placed at the card placement port, the scanning device 14 may be turned on to obtain information and determine whether the type of the placed card is correct. If it is incorrect, according to the strategy, the user is reminded to place the detection card 93 or the balancing card on the card position through the indicator light or the voice, etc., or the user is reminded that there is an error in the placed card.

The present disclosure further provides a multi-flux centrifugal platform 97 that may be applied to the above methods and may be applied to the molecular diagnostic device 100 mentioned above. As illustrated in FIG. 26, FIG. 26 is a structural schematic view of the multi-flux centrifugal platform in some embodiments of the present disclosure. The multi-flux centrifugal platform 97 includes an obtaining module 971, a determining module 972, and a controlling module 973.

The obtaining module 971 is configured to obtain the number of the detection cards 93.

The determining module 972 is configured to determine the strategy for rotating the card holder 65 of the multi-flux centrifugal platform 97 corresponding to the number of the detection cards 93.

The controlling module 973 is configured to control the rotation of the card holder 65 according to the strategy, so that the card position of the card holder 65 is exposed at the card placement port.

In some embodiments, the determining module 972 is configured to perform balance calculation by using the preset algorithm based on the number of the detection cards 93 and the number of the card positions on the card holder 65, so that the strategy is obtained.

In some embodiments, before the determining module 972 determines the strategy for rotating the card holder 65 of the multi-flux centrifugal platform 97 corresponding to the number of the detection cards 93, the determining module 972 is configured to determine the operating mode instruction which is configured for controlling the operation of the multi-flux centrifugal platform last time. The operating mode instruction is the mode instruction that requires the balancing card for balancing or the mode instruction that does not require the balancing card for balancing. Furthermore, the determining module 972 is configured to obtain the strategy based on the number of the detection cards 93 and the operating mode instruction which is configured for controlling the operation of the multi-flux centrifugal platform last time.

In some embodiments, the determining module 972 is configured to determine the operating mode instruction which is configured for controlling the operation of the multi-flux centrifugal platform last time as the mode instruction that requires the balancing card for balancing. Furthermore, the determining module 972 is configured to obtain the sum of the number of the detection cards 93 and the number of the balancing cards on the card holder 65, and configured to obtain the strategy based on the number of the card positions of the card holder 65 and the sum of the number of the detection cards 93 and the number of the balancing cards on the card holder 65. In some embodiments, the determining module 972 is configured to direct perform the balancing based on the number of the detection cards 93, in response to the result that the balancing cannot be performed based on the number of the card positions of the card holder 65 and the sum of the number of the detection cards 93 and the number of the balancing cards on the card holder 65, so that the strategy is obtained. In some embodiments, the controlling module 973 is configured to expose the card position where the balancing card is placed at the card placement port, in response to the result that the balancing cannot be performed based on the number of the card positions of the card holder 65 and the sum of the number of the detection cards 93 and the number of the balancing cards on the card holder 65. In some embodiments, the controlling module 973 is configured to record the current operating mode instruction controlling the operation of the multi-flux centrifugal platform as the mode instruction that does not require the balancing card for balancing, in response to the result that the balancing cannot be performed based on the number of the card positions of the card holder 65 and the sum of the number of the detection cards 93 and the number of the balancing cards on the card holder 65.

In some embodiments, the determining module 972 is configured to perform the balancing based on the number of the card positions on the card holder 65 and the sum of the number of the detecting cards 93 and the number of the balancing cards on the card holder 65, in response to the result that the balancing is able to be performed based on the number of the card positions on the card holder 65 and the sum of the number of the detecting cards 93 and the number of the balancing cards on the card holder 65, so that the strategy is obtained. Furthermore, the controlling module 973 is configured to control the card holder 65 to rotate according to the strategy in response to the initial position of the card position corresponding to the balancing card not being at the card placement port, so that the card position corresponding to the balancing card skips the card placement port, and the empty card position of the card holder 65 is exposed at the card placement port.

In some embodiments, the determining module 972 is configured to determine the operating mode instruction which is configured for controlling the operation of the multi-flux centrifugal platform last time as the mode instruction that does not require the balancing card for balancing. Furthermore, the determining module 972 is configured to obtain the strategy based on the number of the detection cards 93 and the number of the card positions of the card holder 65. In some embodiments, the determining module 972 is configured to perform the balancing by using the balancing cards, in response to the result that the balancing cannot be performed based on the number of the detection cards 93 and the number of the balancing cards on the card holder 65, so that the strategy is obtained.

In some embodiments, the controlling module 973 controls the card holder 65 to rotate according to the strategy. Before the card position of the card holder 65 is exposed at the card placement port, the determining module 972 is configured to determine the current operating mode instruction controlling the operation of the multi-flux centrifugal platform as the mode instruction that requires the balancing card for balancing. Furthermore, after the controlling module 973 controls the card holder 65 to rotate according to the strategy, so that the card position of the card holder 65 is exposed at the card placement port, the controlling module 973 is configured to rotate the card position of the card holder 65 corresponding to the balancing card to the designated position for resetting. In some embodiments, the controlling module 973 is configured to rotate the card position without the balancing card in the card holder 65 to the card placement port, so as to complete the reset.

The present disclosure further provides a computer-readable storage medium. As illustrated in FIG. 27, FIG. 27 is a block schematic view of the computer-readable storage medium in some embodiments of the present disclosure. The computer-readable storage medium 98 stores a computer program 981. When the computer program 981 is executed by a processor, an automatic balancing method based on the multi-flux centrifugal platform in the detection card placement process mentioned above is implemented.

The computer-readable storage medium 98 may be a media that may store program instructions, such as a USB flash disk, a mobile hard disk, a read-only memory (ROM), a random access memory (RAM), a disk, or compact disc. The computer-readable storage medium 98 may also be a server that stores the program instructions. The server may send the stored program instructions to other devices for execution, or the server may run the stored program instructions itself.

In the embodiments provided by the present disclosure, it should be understood that, the disclosed methods and devices may be implemented in other manners. For example, the device embodiments described above are only illustrative. For example, the division of the modules or units is only a logical function division. In actual implementation, other division manners may be possible. For example, a plurality of units or components may be combined or integrated into another system, or some features may be omitted or may not be implemented.

The units described as separate components may or may not be physically separated, and the components displayed as units may or may not be physical units. That is, they may be located in one place, or may also be distributed on a plurality of network units. Some or all of the units may be selected according to actual requirements to implement the solutions of the embodiments.

In addition, the respective functional units in various embodiments of the present disclosure may be integrated into one processing unit, or each unit may exist alone physically, or two or more units may be integrated into one unit. The above-mentioned integrated unit may be realized in the form of hardware or in the form of a software functional unit.

The above descriptions are only some embodiments of the present disclosure, and are not intended to limit the scope of the present disclosure. Any equivalent structure or equivalent flow transformation made by using the contents of the specification and accompanying drawings of the present disclosure, or directly or indirectly applied to other related technical fields, is included in the scope of the patent protection of the present disclosure.

## Claims

1. A control method based on a multi-flux centrifugal platform, comprising:
obtaining the number of detection cards;
determining a strategy for rotating a card holder of the multi-flux centrifugal platform, wherein the strategy corresponds to the number of the detection cards; and
controlling the card holder to rotate according to the strategy, so that a card position of the card holder is exposed at a card placement port.

2. The method according to claim 1, wherein the determining a strategy for rotating a card holder of the multi-flux centrifugal platform, comprises:
performing balance calculation by using a preset algorithm based on the number of the detection cards and the number of the card positions on the card holder, so that the strategy is obtained.

3. The method according to claim 1, wherein
before the determining a strategy for rotating a card holder of the multi-flux centrifugal platform, the method further comprises:
determining an operating mode instruction which configured for controlling operation of the multi-flux centrifugal platform last time, wherein the operating mode instruction is a first mode instruction that requires a balancing card for balancing or a second mode instruction that does not require the balancing card for balancing;
the determining a strategy for rotating a card holder of the multi-flux centrifugal platform, comprises:
obtaining the strategy based on the number of the detection cards and the operating mode instruction which is configured for controlling the operation of the multi-flux centrifugal platform last time.

4. The method according to claim 3, wherein
the determining an operating mode instruction which configured for controlling operation of the multi-flux centrifugal platform last time, comprises:
determining the operating mode instruction which is configured for controlling the operation of the multi-flux centrifugal platform last time as the first mode instruction that requires the balancing card for balancing;
the obtaining the strategy based on the number of the detection cards and the operating mode instruction which is configured for controlling the operation of the multi-flux centrifugal platform last time, comprises:
obtaining a sum of the number of the detection cards and the number of the balancing cards on the card holder; and
obtaining the strategy based on the sum and the number of the card positions of the card holder.

5. The method according to claim 4, wherein the obtaining the strategy based on the sum and the number of the card positions of the card holder, comprises:
directly performing balancing based on the number of the detection cards so that the strategy is obtained, in response to a result that the balancing cannot be performed based on the sum and the number of the card positions of the card holder.

6. The method according to claim 5, wherein the controlling the card holder to rotate according to the strategy, so that a card position of the card holder is exposed at a card placement port, comprises:
exposing the card position where the balancing card is configured to be placed at the card placement port, in response to the result that the balancing cannot be performed based on the sum and the number of the card positions of the card holder.

7. The method according to any one of claims 4-6, wherein the method further comprises:
recording a current operating mode instruction controlling the operation of the multi-flux centrifugal platform as the second mode instruction that does not require the balancing card for balancing, in response to the result that the balancing cannot be performed based on the sum and the number of the card positions of the card holder.

8. The method according to claim 4, wherein
the obtaining the strategy based on the sum and the number of the card positions of the card holder, comprises:
performing the balancing based on the sum and the number of the card positions of the card holder so that the strategy is obtained, in response to a result that the balancing is able to be performed based on the sum and the number of the card positions of the card holder;
the controlling the card holder to rotate according to the strategy, so that a card position of the card holder is exposed at a card placement port, comprises:
controlling the card holder to rotate according to the strategy in response to an initial position of the card position corresponding to the balancing card not being at the card placement port, so that the card position corresponding to the balancing card skips the card placement port, and an empty card position of the card holder is exposed at the card placement port.

9. The method according to claim 3, wherein
the determining an operating mode instruction which configured for controlling operation of the multi-flux centrifugal platform last time, comprises:
determining the operating mode instruction which is configured for controlling the operation of the multi-flux centrifugal platform last time as the second mode instruction that does not require the balancing card for balancing;
the obtaining the strategy based on the number of the detection cards and the operating mode instruction which is configured for controlling the operation of the multi-flux centrifugal platform last time, comprises:
obtaining the strategy based on the number of the detection cards and the number of the card positions of the card holder.

10. The method according to claim 9, wherein the balancing is performed by using the balancing card so that the strategy is obtained, in response to a result that the balancing cannot be performed based on the number of the detection cards and the number of the card positions of the card holder.

11. The method according to claim 9, wherein
before the controlling the card holder to rotate according to the strategy, so that a card position of the card holder is exposed at a card placement port, the method further comprises:
determining a current operating mode instruction controlling the operation of the multi-flux centrifugal platform as the first mode instruction that requires the balancing card for balancing;
after the controlling the card holder to rotate according to the strategy, so that a card position of the card holder is exposed at a card placement port, the method comprises:
rotating the card position of the card holder corresponding to the balancing card to a designated position for resetting.

12. The method according to claim 11, wherein the rotating the card position of the card holder corresponding to the balancing card to a designated position for resetting, comprises:
rotating the card position without the balancing card in the card holder to the card placement port.

13. A multi-flux centrifugal platform, comprising:
an obtaining module, configured to obtain the number of detection cards;
a determining module, configured to determine a strategy for rotating a card holder of the multi-flux centrifugal platform, wherein the strategy corresponds to the number of the detection cards; and
a controlling module, configured to control rotation of the card holder according to the strategy, so that a card position of the card holder is exposed at a card placement port.

14. The multi-flux centrifugal platform according to claim 13, wherein the determining module is configured to perform balance calculation by using a preset algorithm based on the number of the detection cards and the number of the card positions of the card holder, so that the strategy is obtained.

15. The multi-flux centrifugal platform according to claim 13, wherein before the determining module determines the strategy for rotating the card holder of the multi-flux centrifugal platform, the determining module is configured to determine an operating mode instruction which configured for controlling operation of the multi-flux centrifugal platform last time, and the operating mode instruction is a first mode instruction that requires a balancing card or a second mode instruction that does not require a balancing card for balancing; and
the determining module is configured to obtain the strategy based on the number of the detection cards and the operating mode instruction which is configured for controlling the operation of the multi-flux centrifugal platform last time.

16. The multi-flux centrifugal platform according to claim 15, wherein the determining module is configured to determine the operating mode instruction which is configured for controlling the operation of the multi-flux centrifugal platform last time as the first mode instruction that requires the balancing card for balancing; and
the determining module is configured to obtain a sum of the number of the detection cards and the number of the balancing cards on the card holder, and configured to obtain the strategy based on the sum and the number of the card positions of the card holder.

17. The multi-flux centrifugal platform according to claim 16, wherein the determining module is configured to direct perform the balancing based on the number of the detection cards, in response to a result that the balancing cannot be performed based on the sum and the number of the card positions of the card holder.

18. The multi-flux centrifugal platform according to claim 17, wherein the controlling module is configured to expose the card position where the balancing card is placed at the card placement port, in response to the result that the balancing cannot be performed based on the sum and the number of the card positions of the card holder.

19. The multi-flux centrifugal platform according to any one of claims 16-18, wherein the controlling module is configured to record a current operating mode instruction controlling the operation of the multi-flux centrifugal platform as the second mode instruction that does not require the balancing card for balancing, in response to the result that the balancing cannot be performed based on the sum and the number of the card positions of the card holder.

20. A computer-readable storage medium storing a computer program, wherein when the computer program is executed by a processor, a control method based on a multi-flux centrifugal platform is implemented, and the method comprising:
obtaining the number of detection cards;
determining a strategy for rotating a card holder of the multi-flux centrifugal platform, wherein the strategy corresponds to the number of the detection cards; and
controlling the card holder to rotate according to the strategy, so that a card position of the card holder is exposed at a card placement port.
